# EUROPEAN PATENT SPECIFICATION

(11) **EP 2 521 561 B1**
(45) Date of publication and mention of the grant of the patent: **26.07.2017**
(21) Application number: 10782076.3
(22) Date of filing: 03.11.2010
(51) Int. Cl.: A61K 38/10, A61P 1/10

(54) **LINACLOTIDE FOR THE TREATMENT OF CHRONIC CONSTIPATION**
LINACLOTID ZUR BEHANDLUNG VON CHRONISCHER VERSTOPFUNG
LINACLOTIDE POUR LE TRAITEMENT DE LA CONSTIPATION CHRONIQUE

(30) Priority: 30.04.2010 US 330124 P; 18.10.2010 US 394181 P; 03.11.2009 US 257463 P; 22.04.2010 US 327094 P; 03.11.2009 US 257465 P
(43) Date of publication of application: 14.11.2012
(73) Proprietor: Ironwood Pharmaceuticals, Inc., Cambridge, Massachusetts A 02142 (US); Forest Laboratories Holdings Limited, Hamilton HM12 (BM)
(72) Inventor: JOHNSTON, Jeffrey, Boston, MA 02118 (US); FRETZEN, Angelika, Somerville, MA 02143 (US); WITOWSKI, Steven, Melrose, MA 02176 (US); GROSSI, Alfredo, Somerville, MA 02145 (US); ZHAO, Hong, Lexington, MA 02121 (US); LAVINS, Bernard, Joseph, Lexington, MA 02421 (US); DEDHIYA, Mahendra, Pomona, NY 10970-3219 (US); SCHNEIER, Harvey, New York, NY 10013 (US)
(74) Representative: Simpson, Tobias Rutger
(86) International application number: PCT/US2010/055270
(87) International publication number: WO 2011/056850

(56) References cited:
- WO-A2-2010/019266
- JOHNSTON J M ET AL: "T1817 Results From the Randomized Withdrawal Period of a Phase 3 Clinical Trial of Linaclotide in Chronic Constipation", GASTROENTEROLOGY, ELSEVIER, PHILADELPHIA, PA, vol. 138, no. 5, 1 May 2010 (2010-05-01), pages S-585, XP027025221, ISSN: 0016-5085 [retrieved on 2010-04-27]
- CARSON R T ET AL: "Effect of Linaclotide on Quality of Life in Adults With Chronic Constipation: Results From 2 Randomized, Double-Blind, Placebo-Controlled Phase III Trials", GASTROENTEROLOGY, ELSEVIER, PHILADELPHIA, PA, vol. 139, no. 1, 1 July 2010 (2010-07-01), page E19, XP027202951, ISSN: 0016-5085 [retrieved on 2010-07-01]
- ANDRESEN ET AL: "Effect of 5 Days Linaclotide on Transit and Bowel Function in Females With Constipation-Predominant Irritable Bowel Syndrome", GASTROENTEROLOGY, ELSEVIER, PHILADELPHIA, PA, vol. 133, no. 3, 1 September 2007 (2007-09-01), pages 761-768, XP022246622, ISSN: 0016-5085, DOI: DOI:10.1053/J.GASTRO.2007.06.067
- LEMBO A J ET AL: "Linaclotide Significantly Improved Bowel Habits and Relieved Abdominal Symptoms in Adults with Chronic Constipation: Data from a Large 4-Week, Randomized, Double-Blind, Placebo-Controlled Study", GASTROENTEROLOGY, ELSEVIER, PHILADELPHIA, PA, vol. 135, no. 1, 1 July 2008 (2008-07-01), page 295, XP022823131, ISSN: 0016-5085, DOI: DOI:10.1053/J.GASTRO.2008.05.062 [retrieved on 2008-07-01]
- LEMBO A J ET AL: "157 Effect of Linaclotide On IBS-C Symptoms in the First Week of Treatment: Results from a Phase 2B Study", GASTROENTEROLOGY, ELSEVIER, PHILADELPHIA, PA, vol. 136, no. 5, 1 May 2009 (2009-05-01), pages A-30, XP026110687, ISSN: 0016-5085, DOI: DOI:10.1016/S0016-5085(09)60140-2 [retrieved on 2009-05-01]
- VIOLA ANDRESEN ET AL: "Linaclotide Acetate", DRUGS OF THE FUTURE, vol. 33, no. 7, 1 January 2008 (2008-01-01), pages 570-576, XP002576431, ISSN: 0377-8282, DOI: DOI:10.1358/DOF.2008.033.07.1214164
- LAVINS B J ET AL: "418 Effect of Linaclotide On Quality of Life in Adults with Chronic Constipation: Results from a Phase 2B Study", GASTROENTEROLOGY, ELSEVIER, PHILADELPHIA, PA, vol. 136, no. 5, 1 May 2009 (2009-05-01), pages A-71, XP026110867, ISSN: 0016-5085, DOI: DOI:10.1016/S0016-5085(09)60320-6 [retrieved on 2009-05-01]
- HARRIS L A ET AL: "Drug evaluation:Linaclotide, a new direction in the treatment of irritable bowel syndrome and chronic constipation", CURRENT OPINION IN MOLECULAR THERAPEUTICS, CURRENT DRUGS, LONDON, GB, vol. 9, no. 4, 1 August 2007 (2007-08-01), pages 403-410, XP008097427, ISSN: 1464-8431
- JOHNSTON J M ET AL: "Pilot study on the effect of linaclotide in patients with chronic constipation", AMERICAN JOURNAL OF GASTROENTEROLOGY 2009 NATURE PUBLISHING GROUP GBR LNKD- DOI:10.1038/AJG.2008.59, vol. 104, no. 1, January 2009 (2009-01), pages 125-132, XP009149330, ISSN: 0002-9270
- JOHNSTON J M ET AL: "T1817 Results From the Randomized Withdrawal Period of a Phase 3 Clinical Trial of Linaclotide in Chronic Constipation", GASTROENTEROLOGY, ELSEVIER, AMSTERDAM, NL, vol. 138, no. 5, 1 May 2010 (2010-05-01), pages S-585, XP027025221, ISSN: 0016-5085 [retrieved on 2010-04-27]

### SEQUENCE LISTING

<110> Ironwood Pharmaceuticals, Inc.
<120> Treatment of Chronic Constipation
<130> TBD
<140> TBD
   <141> 2010-11-03
<150> US 61/257,463
   <151> 2009-11-03
<160> 1
<170> PatentIn version 3.5
<210> 1
   <211> 14
   <212> PRT
   <213> Artificial Sequence
<220>
   <223> Synthetically generated peptide
<400> 1
   Cys Cys Glu Tyr Cys Cys Asn Pro Ala Cys Thr Gly Cys Tyr
   1               5                   10

## Description

### FIELD OF THE INVENTION

This invention relates to the use of linaclotide to treat chronic constipation.

### BACKGROUND

As many as 34 million Americans suffer from symptoms associated with chronic constipation (CC) and 8.5 million patients have sought treatment. Patients with CC often experience hard and lumpy stools, straining during defecation, a sensation of incomplete evacuation, and fewer than three bowel movements per week. The discomfort and bloating of CC significantly affects patients' quality of life by impairing their ability to work and participate in typical daily activities.

Chronic constipation in a patient may be defined as the presence of fewer than three bowel movements (BMs) per week and by one or more of the following symptoms for at least 12 weeks, which need not be consecutive, in the 12 months before starting chronic treatment with tegaserod, lubiprostone, polyethylene glycol 3350 or any laxative:
a) Straining during greater than 25% of BMs;
b) Lumpy or hard stool during greater than 25% of BMs; and
c) Sensation of incomplete evacuation during greater than 25% of BMs (see, e.g., Rome II criteria (Drossman, 2000), slightly modified from the original).

Half of CC patients are not satisfied with currently available treatments for CC. Thus, there remains a need for new compounds and methods of treating CC.

### SUMMARY

The present invention provides a pharmaceutical composition for use in a method of treating chronic constipation as defined in the appended claims. Also provided is the use of linaclotide for the manufacture of a pharmaceutical composition for the treatment of chronic constipation as defined in the appended claims.

More generally, the disclosure relates to a method of treating chronic constipation.

In one aspect of the disclosure, the method of treating a patient with chronic constipation includes administering a therapeutically effective dose of linaclotide once a day.

In another aspect, the disclosure relates to a method of optimizing a treatment of chronic constipation in a patient with linaclotide.

In one aspect, the method includes:
a) administering a first therapeutically effective dose of linaclotide once a day;
b) determining whether the patient develops loose stools or diarrhea after treatment with linaclotide;
c) wherein if the patient develops loose stools or diarrhea after one or more days of said administering, administering a second therapeutically effective dose of linaclotide once a day, wherein said second therapeutically effective dose is lower than said first therapeutically effective dose.

In still another aspect, a method of optimizing the treatment with linaclotide of a patient with chronic constipation, includes:
a) administering a first therapeutically effective dose of linaclotide once a day on a first schedule;
b) determining whether the patient develops loose stools or diarrhea after treatment with linaclotide;
c) wherein if the patient develops loose stools or diarrhea after one or more days of said administering, administering a second therapeutically effective dose of linaclotide on a second schedule that is less frequent than the first schedule.

In yet another aspect, a method of optimizing the treatment of a patient with chronic constipation, includes:
a) administering a first dose of linaclotide once a day on a first schedule to the patient; and
b) determining whether the administering increases the number of complete spontaneous bowel movements (CSBMs) by the patient to three or greater CSBMs per week;
c) wherein if the patient has fewer than three CSBMs per week, administering a second dose of linaclotide once a day that is higher than said first dose of linaclotide and/or administering said linaclotide on a second schedule that is more frequent than said first schedule.

In a further aspect, a method of optimizing the treatment of a patient with chronic constipation, includes:
(a) administering an amount of linaclotide once a day on a daily basis to the patient; and
(b) determining whether the patient develops loose stools or diarrhea;
wherein the presence of loose stools or diarrhea indicates a need to decrease the amount of linaclotide subsequently administered to said patient and/or decrease the dosing frequency when linaclotide is subsequently administered to said patient.

In another aspect, a method of optimizing the treatment of a patient with chronic constipation, includes:
(a) administering a first amount of linaclotide once a day at a first frequency to the patient; and
(b) determining whether the administering increases the number of complete spontaneous bowel movements (CSBMs) by the patient to three or greater CSBMs per week;
wherein the patient having fewer than three CSBMs per week indicates a need to increase second dose of linaclotide once a day that is higher than said first dose of linaclotide and/or administering said linaclotide on a second schedule that is more frequent than said first schedule.

In certain embodiments of the foregoing aspects, the methods include administering linaclotide formulation including:
(a) linaclotide or pharmaceutically acceptable salts thereof;
(b) CaCl₂;
(c) L-Leucine; and
(d) Hydroxypropyl Methylcellulose
wherein linaclotide is present in the pharmaceutical composition in an amount between 100 µg to 600 µg and the molar ratio of Ca²⁺:leucine:linaclotide is between 5-100:5-50:1.

In some aspects of the present methods, administering of linaclotide provides sustained relief from symptoms of chronic constipation, sustained relief from symptoms of chronic constipation for at least 16 weeks, sustained relief from symptoms of chronic constipation for at least 1 out of 2 weeks, sustained relief of symptoms of chronic constipation for at least 3 out of 4 weeks, 6 out of 12 weeks, or 9 out of 16 weeks.

Importantly, administration of linaclotide as described herein provides one or more of the following advantages: an increase in the rate, for example frequency, of spontaneous bowel movements (SBMs) and complete spontaneous bowel movements (CSBMs) by the patient compared to said patient prior to treatment with linaclotide; a decrease in bloating, such as abdominal bloating, in said patient compared to said patient prior to treatment with linaclotide; a decrease in the abdominal discomfort in said patient compared to said patient prior to treatment with linaclotide; a decrease in the constipation severity in said patient compared to said patient prior to treatment with linaclotide; an improvement in the stool consistency in said patient compared to said patient prior to treatment with linaclotide, a decrease in the straining during defecation in said patient compared to said patient prior to treatment with linaclotide; an improvement in patient assessment of quality of life with constipation; and discontinuing linaclotide administration did not produce a rebound or exacerbation of chronic constipation symptoms.

Further, said patient may exhibit at least one SBM or CSBM within one week, 72 hours, 48 hours, or 24 hours after the administering of a therapeutically effective amount of linaclotide. Administering of a therapeutically effective amount of linaclotide may also produce an improvement in any of SBM rate, CSBM rate, stool consistency, straining, abdominal discomfort, bloating, constipation severity, or a combination thereof, within one week, 72 hours, 48 hours, or 24 hours post-administration.

### BRIEF DESCRIPTION OF THE DRAWINGS

Figure 1 depicts the Trial 2 study design.
Figure 2 depicts the results and improvements of the weekly CSBM rate from Trial 2 for both the treatment period and randomized withdrawal period.
Figure 3 depicts the results and improvements of the weekly SBM rate from Trial 2 for both the treatment period and randomized withdrawal period.
Figure 4 depicts the results and improvements of the weekly SBM rate from Trial 1 and 2 during the treatment periods for placebo, 133 µg linaclotide and 266 µg linaclotide.
Figure 5 depicts the results and qualitative improvement of stool consistency during the 12 week administration of linaclotide in Trials 1 and 2 as described herein.
Figure 6 depicts the results and qualitative improvement in the severity of straining during defecation for the 12 week administration of linaclotide in Trials 1 and 2.
Figure 7 depicts the results and qualitative improvement of bloating during the 12 week administration of linaclotide in Trials 1 and 2.
Figure 8 depicts the results and qualitative improvement of abdominal discomfort during the 12 week administration of linaclotide in Trials 1 and 2.
Figure 9 depicts the results and qualitative improvement of constipation severity during the 12 week administration of linaclotide in Trials 1 and 2.
Figure 10 depicts the results and qualitative improvement in PAC-QOL during the 12 week administration of linaclotide in Trials 1 and 2.
Figure 11 depicts the results of the CSBM overall responder rate of patients in at least six out of 12 weeks with an increase of ≥1 from baseline in weekly CSBMs for both Trial 1 and 2.
Figure 12a depicts the percentages of patients in Trials 1 and 2 reporting at least one SBM within 24 hours of initial dose of the initial treatment with placebo, 133 µg, or 266 µg linaclotide; Figure 12b depicts the percentages of patients reporting at least one CSBM within 24 hours of initial dose.
Figure 13 depicts an example of an analysis of a sample containing linaclotide by HPLC.

These figures are provided by way of examples and are not intended to limit the scope of the present invention.

### DETAILED DESCRIPTION

### Definitions

As used herein, the term "binder" refers to any pharmaceutically acceptable binder that may be used in the practice of the disclosure. Examples of pharmaceutically acceptable binders include, without limitation, a starch (e.g., corn starch, potato starch and pre-gelatinized starch (e.g., STARCH 1500® and STARCH 1500 LM®, sold by Colorcon, Ltd. and other starches), maltodextrin, gelatin, natural and synthetic gums such as acacia, powdered tragacanth, guar gum, cellulose and its derivatives (e.g., methylcellulose, hydroxyethyl cellulose, hydroxyethyl methylcellulose, hydroxypropyl cellulose and hydroxypropyl methylcellulose (hypromellose), ethyl cellulose, cellulose acetate, carboxymethyl cellulose calcium, sodium carboxymethyl cellulose, carboxymethylcellulose, microcrystalline cellulose (e.g., AVICEL™, such as, AVICEL-PH-101™, -103™ and - 105™, sold by FMC Corporation, Marcus Hook, PA, USA)), polyvinyl alcohol, polyvinyl pyrrolidone (e.g., polyvinyl pyrrolidone K30), and mixtures thereof.

As used herein, the term "filler" refers to any pharmaceutically acceptable filler that may be used in the practice of the disclosure. Examples of pharmaceutically acceptable fillers include, without limitation, talc, calcium carbonate (e.g., granules or powder), dibasic calcium phosphate, tribasic calcium phosphate, calcium sulfate (e.g., granules or powder), microcrystalline cellulose (e.g., Avicel PH101 or Celphere CP-305), powdered cellulose, dextrates, kaolin, mannitol, silicic acid, sorbitol, starch (e.g., Starch 1500), pre-gelatinized starch, lactose, glucose, fructose, galactose, trehalose, sucrose, maltose, isomalt, raffinose, maltitol, melezitose, stachyose, lactitol, palatinite, xylitol, myoinositol, and mixtures thereof.

Examples of pharmaceutically acceptable fillers that may be particularly used for coating with linaclotide include, without limitation, talc, microcrystalline cellulose (e.g., Avicel PH101 or Celphere CP-305), powdered cellulose, dextrates, kaolin, mannitol, silicic acid, sorbitol, starch, pre-gelatinized starch, lactose, glucose, fructose, galactose, trehalose, sucrose, maltose, isomalt, dibasic calcium phosphate, raffinose, maltitol, melezitose, stachyose, lactitol, palatinite, xylitol, mannitol, myoinositol, and mixtures thereof.

As used herein, the term "additives" refers to any pharmaceutically acceptable additive. Pharmaceutically acceptable additives include, without limitation, disintegrants, dispersing additives, lubricants, glidants, antioxidants, coating additives, diluents, surfactants, flavoring additives, humectants, absorption promoting additives, controlled release additives, anti-caking additives, anti-microbial agents (e.g., preservatives), colorants, desiccants, plasticizers and dyes.

As used herein, an "excipient" is any pharmaceutically acceptable additive, filler, binder or agent.

As used herein, "spontaneous bowel movement" of SBM, is a bowel movement that occurs in the absence of laxative, enema, or suppository usage within the preceding 24 hours.

As used herein, a "complete spontaneous bowel movement" or CSBM is an SBM that is accompanied by the patient self-reporting a feeling of complete emptying of the bowel.

As used herein, a "CSBM weekly responder" is a patient who had a three or more CSBMs per week and an increase of at least one CSBM per week over baseline.

As used herein, a "12-week CSBM overall responder" is a patient who is a CSBM weekly responder for at least nine of the 12 weeks of the treatment period.

As used herein, "Bristol Stool Form Scale" or BSFS is seven-point scale measuring stool consistency. BSFS is a surrogate marker of gastrointestinal transit time.

As used herein, "PAC-QOL" is the Patient Assessment of Constipation-Quality of Life with 28 questions developed through literature, qualitative interviews, and clinician review. The assessment was grouped into 4 subscales including physical discomfort (4 items); psychosocial discomfort (8 items); worries/concerns (11 items); satisfaction (5 items). Scoring for overall score for PAC-QOL is based on the average of responses to 28 items wherein a lower score represents a higher quality of life (QOL). Subscale scoring is based on the average score of individual items in the subscale wherein a lower score represents a higher quality of life (QOL).

As used herein, "rebound" is the exacerbation of the severity of a symptom experienced by a patient after discontinuation of a treatment as compared to the severity of the symptom experienced by the patient prior to that treatment.

As used herein, "rapid relief" is the improvement of one or more symptoms described herein within one week of initiating a treatment as described herein.

### Guanylate Cyclase

Guanylate cyclase C (GC-C) is a transmembrane receptor that is located on the apical surface of epithelial cells in the stomach and intestine. The receptor has an extracellular ligand-binding domain, a single transmembrane region and a C-terminal guanylyl cyclase domain. When a ligand binds to the extracellular domain of GC-C, the intracellular catalytic domain catalyzes the production of cGMP from GTP. *In vivo,* this increase in intracellular cGMP initiates a cascade of events that leads to increased secretion of chloride and bicarbonate into the intestinal lumen, increased luminal pH, decreased luminal sodium absorption, increased fluid secretion, and acceleration of intestinal transit. cGMP, which is secreted bidirectionally from the epithelium into the mucosa and lumen, has also been shown to dampen afferent C fiber firing, suggesting a potential mechanism for the observed analgesic effects of GC-C agonists on visceral pain.

Linaclotide is a peptide GC-C agonist that is orally administered and currently in clinical trials for treatment of irritable bowel syndrome with constipation (IBS-c) and CC. In Phase 2b studies for CC, linaclotide reduced constipation, abdominal discomfort, and bloating throughout the four-week treatment period. Orally administered linaclotide acts locally by activating GC-C at the luminal surface; there are no detectable levels of linaclotide seen systemically after oral administration at therapeutic dose levels.

Linaclotide is a 14 amino acid peptide having the sequence Cys₁ Cys₂ Glu₃ Tyr₄ Cys₅ Cys₆ Asn₇ Pro₈ Ala₉ Cys₁₀ Thr₁₁ Gly₁₂ Cys₁₃ Tyr₁₄ with disulfide bonds between Cys₁ and Cys₆, between Cys₂ and Cys₁₀ and between Cys₅ and Cys₁₃.

The dose range of linaclotide for adult humans is generally from 25 µg to 6 mg per day orally. In a further embodiment, the dose range is 25 µg to 2 mg per day orally. In some embodiments, the dose range for adult humans is 50 µg to 1 mg per day orally (e.g., 50 µg, 67.5 µg, 100 µg, 133 µg, 150 µg, 200 µg, 250 µg, 266 µg, 300 µg, 350 µg, 400 µg, 450 µg, 500 µg, 550 µg, 600 µg, 650 µg, 700 µg, 750 µg, 800 µg, 850 µg, 900 µg, 950 µg or 1 mg). In further embodiments, the dose range is 100 µg to 600 µg per day orally. In other embodiments, the dose is 50 µg, 67.5 µg, 100 µg, 133 µg, 150 µg, 200 µg, 266 µg, 300 µg, 400 µg, 500 µg or 600 µg linaclotide per day orally.

### Administration of peptides and GC-C receptor agonists

For treatment of gastrointestinal disorders, the peptides and agonists of the disclosure are preferably administered orally, e.g., as a tablet, gel, paste, sachet, a pellet, a capsule, a slurry, a liquid, a powder or in some other form. Orally administered compositions can include, for example, binders, lubricants, inert diluents, lubricating, surface active or dispersing additives, flavoring additives, and humectants. Orally administered formulations such as tablets may optionally be coated or scored and may be formulated so as to provide sustained, delayed or controlled release of the linaclotide therein. The linaclotide can be co-administered or co-formulated with other medications. In one embodiment, the linaclotide composition can be co-administered with other medications used to treat gastrointestinal disorders.

In certain embodiments, the linaclotide composition is provided in a unit dosage form. In some embodiments, the unit dosage form is a capsule, a tablet, a sachet, a pellet or a powder. In one such embodiment, the unit dosage form is a capsule or tablet. Such unit dosage forms may be contained in a container such as, without limitation, a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container can be used together in a single package to provide a single dosage form. For example, tablets or capsules may be contained in a bottle which is in turn contained within a box. In some embodiments, the unit dosage forms are provided in a container further comprising a desiccant. In a further embodiment, the unit dosage forms, e.g., a quantity of tablets or capsules, are provided in a container, e.g., a bottle, jar or re-sealable bag, containing a desiccant. In a further embodiment, the container containing the unit dosage forms is packaged with administration or dosage instructions. In certain embodiments, the linaclotide composition is provided in a kit. The linaclotide composition described herein and combination therapy agents can be packaged as a kit that includes single or multiple doses of two or more agents, each packaged or formulated individually, or single or multiple doses of two or more agents packaged or formulated in combination. Thus, the linaclotide composition can be present in first container, and the kit can optionally include one or more agents in a second container. The container or containers are placed within a package, and the package can optionally include administration or dosage instructions.

In various embodiments, the unit dosage form is administered with food at anytime of the day, without food at anytime of the day, with food after an overnight fast (e.g., with breakfast). In various embodiments, the unit dosage form is administered once a day, twice a day or three times a day. The unit dosage form can optionally comprise other additives. In some embodiments, one, two or three unit dosage forms will contain the daily oral dose of linaclotide. The precise amount of compound administered to a patient will be the responsibility of the attendant physician. However, the dose employed will depend on a number of factors, including the age and sex of the patient, the precise disorder being treated, and its severity.

### Methods of Treating Chronic Constipation

In some aspects, the disclosure provides a method of treating a patient with chronic constipation, comprising administering a therapeutically effective dose of linaclotide once a day.

In some embodiments, the therapeutically effective dose is administered once a day in the morning.

In other embodiments, the therapeutically effective dose is administered at least 30 minutes before ingestion of food.

In still other embodiments, the therapeutically effective dose is 100 to 600 µg linaclotide. For instance, in some specific embodiments, the therapeutically effective dose is 133 µg or 266 µg of linaclotide.

In some embodiments, the method includes administering linaclotide for a period of greater than four weeks. In some embodiments, the method includes administering linaclotide for a period of greater than six weeks. For instance, in some specific embodiments, linaclotide is administered for a period of at least 6 weeks.

In some embodiments, linaclotide is administered for a period of at least 12 weeks.

In some instances, linaclotide is administered each day of the week. In other instances, linaclotide is administered at least once a week, at least twice a week, at least three times a week, at least four times a week, at least five times a week or at least six times a week.

In some aspects, the method of treating chronic constipation includes administering a formulation containing:
(a) linaclotide or pharmaceutically acceptable salts thereof;
(b) CaCl₂;
(c) L-Leucine; and
(d) Hydroxypropyl Methylcellulose
wherein linaclotide is present in the pharmaceutical composition in an amount between 100 µg to 600 µg and the molar ratio of Ca²⁺:leucine:linaclotide is between 5-100:5-50:1.

In some embodiments, the linaclotide formulation is provided as a capsule or tablet. In some instances, the linaclotide formulation is provided as a capsule. In some specific instances, linaclotide is present in the tablet or capsule in an amount of 133 or 266 µg. In some embodiments, CaCl₂ is present in the tablet or capsule in an amount of 1541 µg. In some embodiments, leucine is present in the tablet or capsule in an amount of 687 µg. In some embodiments, hydroxypropyl methylcellulose is present in the tablet or capsule in an amount of 700 µg.

In some aspects, the method of treating chronic constipation provides an increase in the number of complete spontaneous bowel movements (CSBMs) by the patient to three or greater CSBMs per week.

In some embodiments, administering linaclotide according to the disclosure increases the number of CSBMs by the patient by at least one CSBM per week compared to said patient prior to treatment with linaclotide.

In still further embodiments, administering linaclotide according to the disclosure increases the number of CSBMs by the patient to three or greater CSBMs per week.

In other embodiments, administering linaclotide according to the disclosure increases the number of CSBMs by the patient by at least one CSBM per week compared to the number of CSBMs by said patient prior to treatment with linaclotide.

In some embodiments, the method of treating chronic constipation according to the disclosure results in a 1.5 or greater fold increase of the 12 week CSBM overall responder rate compared to baseline, i.e., prior to treatment with linaclotide. In other embodiments, the fold increase of the 12 week CSBM overall responder rate is 2.0 or greater. In other embodiments, the fold increase of the 12 week CSBM overall responder rate is 2.5 or greater. In still other embodiments, the fold increase of the 12 week CSBM overall responder rate is 3.0 or greater. In other embodiments, the fold increase of the 12 week CSBM overall responder rate is 3.5 or greater. In other embodiments, the fold increase of the 12 week CSBM overall responder rate is 4.0 or greater. In other embodiments, the fold increase of the 12 week CSBM overall responder rate is 4.5 or greater. In other embodiments, the fold increase of the 12 week CSBM overall responder rate is 5.0 or greater. In other embodiments, the fold increase of the 12 week CSBM overall responder rate is 5.5 or greater. In other embodiments, the fold increase of the 12 week CSBM overall responder rate is 6.0 or greater. In other embodiments, the fold increase of the 12 week CSBM overall responder rate is 6.5 or greater.

In some embodiments, the method of treating chronic constipation according to the disclosure provides a patient a 1.5 or greater fold increase in that patient's 12 week CSBM overall response compared to baseline, i.e., prior to treatment with linaclotide. In other embodiments, the fold increase in that patient's 12 week CSBM overall response is 2.0 or greater. In other embodiments, the fold increase in that patient's 12 week CSBM overall response is 2.5 or greater. In still other embodiments, the fold increase in that patient's 12 week CSBM overall response is 3.0 or greater. In other embodiments, the fold increase in that patient's 12 week CSBM overall response is 3.5 or greater. In other embodiments, the fold increase in that patient's 12 week CSBM overall response is 4.0 or greater. In other embodiments, the fold increase in that patient's 12 week CSBM overall response is 4.5 or greater. In other embodiments, the fold increase in that patient's 12 week CSBM overall response is 5.0 or greater. In other embodiments, the fold increase in that patient's 12 week CSBM overall response is 5.5 or greater. In other embodiments, the fold increase in that patient's 12 week CSBM overall response is 6.0 or greater. In other embodiments, the fold increase in that patient's 12 week CSBM overall response is 6.5 or greater.

In some embodiments, administering linaclotide according to the disclosure results in a 1.0 or greater increase from baseline in the average weekly CSBMs. In other embodiments, the increase in average weekly CSBMs from baseline is 1.5 or greater. In other embodiments, the increase in average weekly CSBMs from baseline is 2.0 or greater. In other embodiments, the increase in average weekly CSBMs from baseline is 2.5 or greater. In other embodiments, the increase in average weekly CSBMs from baseline is 3.0 or greater.

In still other embodiments, administering linaclotide according to the disclosure results in a 1.0 or greater increase from baseline in the weekly CSBMs in at least three-fourths of the weeks for which the therapy is administered. In other embodiments, the administration results in a 1.5 or greater increase in weekly CSBMs from baseline in at least three-fourths of the weeks for which the therapy is administered. In other embodiments, the administration results in a 2.0 or greater increase in weekly CSBMs from baseline in at least three-fourths of the weeks for which the therapy is administered. In other embodiments, the administration results in a 2.5 or greater increase in weekly CSBMs from baseline in at least three-fourths of the weeks for which the therapy is administered. In other embodiments, the administration results in a 3.0 or greater increase in weekly CSBMs from baseline in at least three-fourths of the weeks for which the therapy is administered.

In some embodiments, administering linaclotide according to the disclosure results in a 1.0 or greater increase from baseline in the average weekly SBMs. In other embodiments, the increase in average weekly SBMs from baseline is 1.5 or greater. In other embodiments, the increase in average weekly SBMs from baseline is 2.0 or greater. In other embodiments, the increase in average weekly SBMs from baseline is 2.5 or greater. In other embodiments, the increase in average weekly SBMs from baseline is 3.0 or greater.

In some embodiments, administering linaclotide to a patient according to the disclosure results in a mean increase of 1 or greater from baseline in weekly CSBMs. In some embodiments, administering linaclotide to a patient according to the disclosure results in a mean increase of 2 or greater from baseline in weekly CSBMs.

In some further aspects, the method of treating chronic constipation according to the disclosure decreases bloating in said patient compared to said patient prior to treatment with linaclotide. In some embodiments, the bloating is abdominal bloating.

In some embodiments, the method of treating chronic constipation according to the disclosure decreases abdominal discomfort in the patient compared to said patient prior to treatment with linaclotide.

In other embodiments, the method of treating chronic constipation according to the disclosure decreases constipation severity in the patient compared to said patient prior to treatment with linaclotide.

In yet some further embodiments, the method of treating chronic constipation according to the disclosure improves stool consistency in the patient compared to said patient prior to treatment with linaclotide.

In other embodiments, the method of treating chronic constipation according to the disclosure decreases straining during defecation in the patient compared to said patient prior to treatment with linaclotide.

In other embodiments, the method of treating chronic constipation according to the disclosure includes improved patient assessment of constipation quality of life as compared to prior treatment with linaclotide.

In still further embodiments, the method of treating chronic constipation according to the disclosure improves at least two symptoms in a patient compared to said symptoms prior to linaclotide treatment, wherein the symptoms are selected from an increase in the number of CSBMs per week, a decrease in bloating, a decrease in abdominal discomfort, a decrease in constipation severity, an improvement in stool consistency or a decrease in straining during defecation. For instance, in some embodiments, administering linaclotide further increases the number of CSBMs by the patient to three or greater CSBMs per week.

In some embodiments, the method of treating a patient with chronic constipation comprises administering a therapeutically effective dose of linaclotide, wherein linaclotide produces a rapid or sustained relief of symptoms associated with chronic constipation.

In some embodiments, the patient has at least one SBM or CSBM within one week after the administering of a therapeutically effective amount of linaclotide, within 72 hours after the administering of a therapeutically effective amount of linaclotide, within 48 hours after the administering of a therapeutically effective amount of linaclotide, or within 24 hours after the administering of a therapeutically effective amount of linaclotide.

In some embodiments, the method of treating chronic constipation according to the disclosure may improve SBM rate, CSBM rate, stool consistency, straining, abdominal discomfort, bloating, constipation severity, or a combination thereof, within one week, 72 hours, 48 hours, 24 hours after the administering of a therapeutically effective amount of linaclotide.

In another aspect, the method of treating chronic constipation according to the disclosure includes the absence of a symptom rebound when discontinuing the administration of a therapeutically effective dose of linaclotide.

In other embodiments, the method of treating chronic constipation according to the disclosure includes the absence of a symptom rebound when discontinuing the administration of a therapeutically effective dose of linaclotide, wherein said symptoms are selected from a decrease in the rate of CSBMs per week, a decrease in the rate of SBMs per week, an increase in bloating, an increase in abdominal discomfort, an increase in constipation severity, a decrease in stool consistency, or an increase in straining during defecation.

In further embodiments, discontinuing the administration of linaclotide does not produce a symptom rebound of weekly CSBMs for a patient.

In other embodiments, discontinuing the administration of linaclotide does not produce a symptom rebound of weekly SBMs for a patient.

In still other embodiments, discontinuing the administration of linaclotide does produce a symptom rebound of stool consistency for a patient.

In some embodiments, discontinuing the administration of linaclotide does not produce a symptom rebound of severity of straining during defecation for a patient.

In some embodiments, discontinuing the administration of linaclotide does not produce a symptom of abdominal discomfort for a patient.

In other embodiments, discontinuing the administration of linaclotide does not produce a symptom rebound of bloating for a patient.

In some embodiments, discontinuing the administration of linaclotide does not produce a symptom rebound of constipation severity for a patient.

In still further embodiments, discontinuing the administration of linaclotide does not produce a symptom rebound for global relief of constipation for a patient.

In other aspects, the disclosure provides a method of optimizing the treatment with linaclotide of a patient with chronic constipation. The method includes:
a) administering a first therapeutically effective dose of linaclotide once a day;
b) determining whether the patient develops loose stools or diarrhea after treatment with linaclotide;
c) wherein if the patient develops loose stools or diarrhea after one or more days of said administering, administering a second therapeutically effective dose of linaclotide once a day, wherein said second therapeutically effective dose is lower than said first therapeutically effective dose.

In some embodiments, the first therapeutically effective dose of linaclotide is 266 µg and the second therapeutically effective dose of linaclotide is 133 µg.

In some further aspects, the disclosure provides a method of optimizing the treatment with linaclotide of a patient with chronic constipation. The method includes:
a) administering a first therapeutically effective dose of linaclotide once a day on a first schedule;
b) determining whether the patient develops loose stools or diarrhea after treatment with linaclotide;
c) wherein if the patient develops loose stools or diarrhea after one or more days of said administering, administering a second therapeutically effective dose of linaclotide on a second schedule that is less frequent than the first schedule.

In some embodiments, the first therapeutically effective dose is the same as the second therapeutically effective dose and the second schedule is less frequent than the first schedule.

In other embodiments, the first therapeutically effective dose and the second therapeutically effective dose are each 266 µg or are each 133 µg.

In still other embodiments, the second therapeutically effective dose is lower than the first therapeutically effective dose. For instance, the first therapeutically effective dose of linaclotide is 266 µg and the second therapeutically effective dose of linaclotide is 133 µg and the first schedule and the second schedule are the same.

In other embodiments, the second schedule that is less frequent than the first schedule.

In still other embodiments, the first schedule is administration of linaclotide on a daily basis.

In yet further embodiments, the second schedule is administration of linaclotide every other day, every third day, every fourth day, every fifth day, every sixth day or once weekly.

In still another aspect, the disclosure provides a method of optimizing the treatment of a patient with chronic constipation. The method includes:
a) administering a first dose of linaclotide once a day on a first schedule to the patient;
b) determining whether the administering increases the number of complete spontaneous bowel movements (CSBMs) by the patient to three or greater CSBMs per week; and
c) wherein if the patient has fewer than three CSBMs per week, administering a second dose of linaclotide once a day that is higher than said first dose of linaclotide and/or administering said linaclotide on a second schedule that is more frequent than said first schedule.

In some embodiments, step (b) further requires determining whether the number of CSBMs by the patient increases by at least one CSBM per week compared to the number of CSBMs by said patient prior to treatment with linaclotide; wherein if said number of CSBMs does not increase by at least one CSBM per week, then administering a second dose of linaclotide once a day that is higher than said first dose of linaclotide and/or administering said linaclotide on a second schedule that is more frequent than said first schedule.

In some embodiments, the first dose is lower than the second therapeutically effective dose.

In specific embodiments, the first dose is 133 µg and the second dose is 266 µg.

In certain embodiments, the first schedule and the second schedule are the same and is administration of linaclotide on a daily basis.

In some embodiments, the first dose and the second dose are the same and the second schedule is more frequent than said first schedule. In some instances, the second schedule is administration of linaclotide on a daily basis. In other instances, the first schedule is administration of linaclotide every other day, every third day, every fourth day, every fifth day, every sixth day or once weekly.

In still further aspects, the disclosure provides a method of optimizing the treatment of a patient with chronic constipation. The method includes:
(a) administering an amount of linaclotide once a day on a daily basis to the patient; and
(b) determining whether the patient develops loose stools or diarrhea;
wherein the presence of loose stools or diarrhea indicates a need to decrease the amount of linaclotide subsequently administered to said patient and/or decrease the dosing frequency when linaclotide is subsequently administered to said patient.

In some embodiments, the amount of linaclotide subsequently administered to said patient is decreased.

In other embodiments, the dosing frequency when linaclotide is subsequently administered is decreased.

In still further aspects, the disclosure provides a method of optimizing the treatment of a patient with chronic constipation. The method includes:
(a) administering a first amount of linaclotide once a day at a first frequency to the patient; and
(b) determining whether the administering increases the number of complete spontaneous bowel movements (CSBMs) by the patient to three or greater CSBMs per week;
wherein the patient having fewer than three CSBMs per week indicates a need to increase second dose of linaclotide once a day that is higher than said first dose of linaclotide and/or administering said linaclotide on a second schedule that is more frequent than said first schedule.

In some embodiments, step (b) further requires determining whether the number of CSBMs by the patient increases by at least one CSBM per week compared to the number of CSBMs by said patient prior to treatment with linaclotide; wherein an increase of less than one CSBM per week indicates a need to increase second dose of linaclotide once a day that is higher than said first dose of linaclotide and/or administering said linaclotide on a second schedule that is more frequent than said first schedule.

In other embodiments, linaclotide can be used to treat bowel symptoms, abdominal symptoms, and rectal symptoms.

In further embodiments, linaclotide can be used to treat bowel symptoms selected from infrequent bowel movements, hard or lumpy stool, straining, stools too small or too large, unsuccessful attempts to have bowel movements, incomplete bowel movements, long duration of bathroom visit, or digital manipulation.

In still further embodiments, linaclotide can be used to treat abdominal symptoms selected from abdominal pain, bloating, abdominal discomfort, stomach pain/aches "belly aches", abdominal cramping, feeling of fullness/feeling "stuffed", feeling back-up "loaded" or impacted, gas, trapped gas, backed up gas, gas pockets, passing gas, gas pain, pain in sides or one side, upset stomach, acid stomach, stomach sour, strong odor of gas, or burping/belching.

In still further embodiments, linaclotide can be used to treat rectal symptoms selected from rectal pain, hemorrhoids, rectal bleeding, rectal tearing, or rectal "burning."

### EXAMPLES

### Example 1: Preparation of Linaclotide

Linaclotide as described herein was prepared by solid phase chemical synthesis and natural folding (air oxidation) by Polypeptide Laboratories (Torrance, CA). The oral linaclotide formulation was prepared by Forest Laboratories, Inc. (New York, NY).

### Example 2: Linaclotide Formulations

The formulations used in the disclosure contain linaclotide or a pharmaceutically acceptable salt of linaclotide. The formulations are stable and have a sufficient shelf life for manufacturing, storing and distributing the drug. For example, the formulations have an expected shelf life of at least 12 months at room temperature storage conditions (e.g., 25°C/60 percent relative humidity (RH)) and up to at least 18 months or 24 months at room temperature storage conditions (e.g., 25°C/60 percent RH). In the formulations, greater than or equal to 95 percent of the original amount of linaclotide in the composition remains after three months when packaged samples are stored at accelerated conditions (40°C/75 percent RH) when assessed in an assay on a weight/weight basis as determined by high pressure liquid chromatography (HPLC) against a linaclotide reference standard.

The GC-C receptor agonist polypeptide formulations are prepared from a solution, e.g., an aqueous solution ("the coating solution"), comprising: (i) a GC-C receptor agonist polypeptide such as linaclotide or a pharmaceutically acceptable salt thereof; (ii) a cation selected from Mg²⁺, Ca²⁺, Zn²⁺, Mn²⁺, K⁺, Na⁺ and Al³⁺ and/or a sterically hindered primary amine (e.g., leucine); and optionally (iii) a pharmaceutically acceptable binder. The GC-C receptor agonist polypeptide formulations can optionally include one or more of a pharmaceutically acceptable glidant, a pharmaceutically acceptable lubricant or a pharmaceutically acceptable additive that acts as both a glidant and lubricant.

It has been found that a cation selected from Mg²⁺, Ca²⁺, Zn²⁺, Mn²⁺, K⁺, Na⁺ and Al³⁺ is useful for suppressing the formation of an oxidation product of the GC-C receptor agonist polypeptide linaclotide during storage. It has also been found that a sterically hindered primary amine is useful for suppressing the formation of a formaldehyde imine adduct of the GC-C receptor agonist polypeptide linaclotide ("formaldehyde imine product") during storage. Thus, the GC-C receptor agonist polypeptide formulations comprising a cation selected from Mg²⁺, Ca²⁺, Zn²⁺, Mn²⁺, K⁺, Na⁺ or Al³⁺-e.g., a divalent cation selected from Zn²⁺, Mg²⁺ and Ca²⁺-and/or a sterically hindered primary amine, such as an amino acid, have a sufficient shelf life (as measured by chromatographic purity and/or by a weight/weight assay) for manufacturing, storing and distributing the drug. Further, while the presence of a sterically hindered amine alone can increase the formation of a hydrolysis product of linaclotide during storage, the combination of a sterically hindered primary amine and a cation, e.g., the combination of leucine and Ca²⁺, suppresses the formation of the hydrolysis product of the GC-C receptor agonist polypeptide as well as the oxidation product of GC-C receptor agonist polypeptide during storage, leading to an even greater overall stability as determined by a weight/weight assay and/or by chromatographic purity.

GC-C receptor agonist polypeptide formulations are typically produced as follows.

### Preparation of the Coating Solution:

Approximately 8.3 kg of purified water is mixed with hydrochloric acid to create a solution with a pH between 1.5 and 2.0. An oxidation-suppressing cation, if used, is added to the solution in a quantity to provide the desired concentration, and the solution is mixed for sufficient time to produce a clear solution. A sterically hindered primary amine, if used, is added to the solution in a quantity to provide the desired concentration, and the solution is mixed for sufficient time to produce a clear solution. Other additives, such as antioxidants, are then added, if desired. The binder is then added to the solution and the solution is mixed for sufficient time to achieve a clear solution. The pH of the solution is tested, and hydrochloric acid is added if necessary to produce a solution having a pH between 1.5 and 2.0. This is Solution 1. Approximately 8.3 kg of purified water is mixed with hydrochloric acid to create a solution with a pH between 1.5 and 2.0. The desired amount of linaclotide is added to the solution and mixed for 10 to 30 minutes. The pH of the solution is tested, and hydrochloric acid is added if necessary to produce a solution having a pH between 1.5 and 2.0. This is Solution 2. Solution 1 and Solution 2 are then mixed together. The pH of the solution is tested, and hydrochloric acid is added if necessary to produce a solution having a pH between 1.5 and 2.0. This is the coating solution.

### Preparation of the Active Beads:

Approximately 24.19 kg of microcrystalline cellulose beads are added to a Wurster Column of a Glatt GPCG-30 Fluid Bed. The microcrystalline cellulose beads are fluidized and heated to product temperature of 45-47°C. Next, the coating solution is layered to the beads. The product spraying temperature is controlled between 37°C and 47°C by controlling inlet temperature, spray rate, atomization pressure, and air volume. After the entire coating solution is layered to the beads, the beads are dried with a product drying temperature of 37°C to 47°C. The product of this process is referred to as active beads.

### Example 3: Measurement of Linaclotide Content and Purity

Linaclotide content and purity, as well as measurement of linaclotide-related substances may be determined, for example, by reverse phase gradient liquid chromatography using an Agilent Series 1100 LC System with Chemstation Rev A.09.03 software or equivalent. A YMC Pro™ C18 column (dimensions: 3.0 x 150 mm, 3.5 um, 120 Å; Waters Corp., Milford, MA) or equivalent is used and is maintained at 40°C. Mobile phase A (MPA) consists of water with 0.1% trifluoroacetic acid while mobile phase B (MPB) consists of 95% acetonitrile:5% water with 0.1% trifluoroacetic acid. Elution of linaclotide and its related substances is accomplished with a gradient from 0% to 47% MPB in 28 minutes followed by a ramp to 100% MPB in 4 minutes with a 5 minute hold at 100% MPB to wash the column. Re-equilibration of the column is performed by returning to 0% MPB in 1 minute followed by a 10 minute hold at 100% MPA. The flow rate is 0.6 mL/min and detection is accomplished by UV at 220 nm.

Samples for analysis are prepared by addition of the contents of linaclotide capsules to 0.1 N HCl to obtain a target concentration of 20 µg linaclotide/mL. A total of 100 µL of this solution is injected onto the column.

Linaclotide content is measured by determining the linaclotide concentration in the prepared sample against a similarly prepared external linaclotide standard.

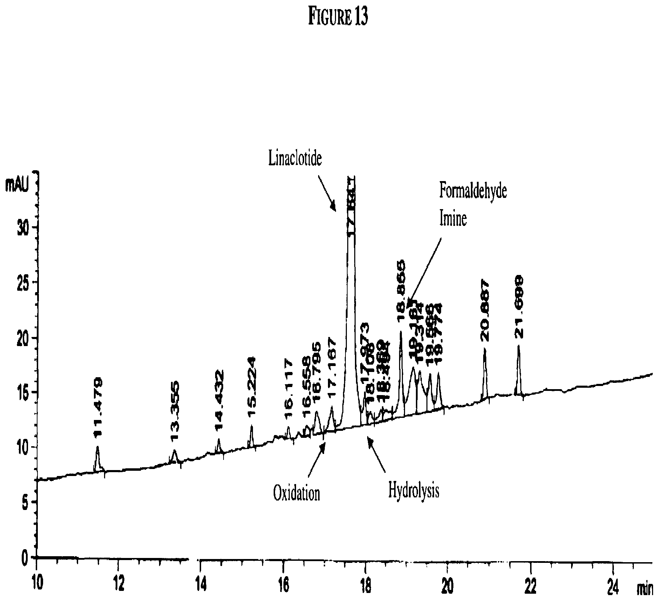

An example of an analysis of linaclotide by HPLC is shown in Figure 13, wherein "Oxidation" refers to the linaclotide oxidation product, "Formaldehyde Imine" refers to the linaclotide formaldehyde imine product and "Hydrolysis" refers to the linaclotide hydrolysis product.

### Example 4: Linaclotide Capsule Formulation

A linaclotide capsule formulation was produced essentially as described in Examples 1 and 2 wherein Table 1 provides the amounts of cation, sterically hindered primary amine, binder, linaclotide and beads, and their theoretical weights (mg/g) and (kg/Batch) for the complete Linaclotide Beads Drug Layer Solution. Table 2 provides the conditions under which the beads were coated. Table 3 provides the ingredients and theoretical weights (mg/g) and (kg/Batch) for the preparation for the Linaclotide Active Beads.

The Linaclotide active beads were tested for linaclotide content. Based on the assay of the active beads, an appropriate amount of active beads was filled into size 2 hard gelatin capsules, (weight 61 mg), using an MG2 Futura encapsulation machine, to achieve the desired linaclotide concentration. The 150 µg linaclotide capsules contained 56 mg linaclotide beads (600 µg linaclotide/225 mg beads) having an effective linaclotide content of 133 µg, while the 300 µg linaclotide capsules contained 113 mg linaclotide beads (600 µg linaclotide/225 mg beads) having an effective linaclotide content of 266 µg. The linaclotide content can be measured, for example, by using the assay described in Example 3 or by other methods.

**Table 1**

| **Ingredients** | **Function** | **Theoretical Weight (mg/g)** | **Theoretical Weight (kg/batch)** |
|---|---|---|---|
| Linaclotide | API | 2.67 | 0.067 |
| CaCl₂•2H₂O, USP, EP, BP, JP | Stabilizer | 15.41 | 0.385 |
| L-Leucine, USP | Stabilizer | 6.87 | 0.172 |
| Hydroxypropyl Methylcellulose, USP (Methocel E5 Premium LV) | Binder | 7.00 | 0.175 |
| Purified Water, USP | -- | -- | 16.666 |
| HCl (36.5-38.0), NF | -- | -- | 0.114 |

**Table 2**

| **Product Spraying Temp (°C)** | **Inlet Temp (°C)** | **Spray rate (g/min)** | **Atomization Pressure (bar)** | **Process Air Volume (cfm)** | **Product Drying Temp (°C)** |
|---|---|---|---|---|---|
| 64.9 - 65.1 | 80 | 150 | 2.0 | 515-564 | 54.9 - 55.0 |

**Table 3**

| **Ingredients** | **Function** | **Theoretical Weight (mg/g)** | **Theoretical Weight (kg/batch)** |
|---|---|---|---|
| Linaclotide Beads Drug Layer Solution | Coating solution | 31.95 | 0.799 |
| Microcrystalline cellulose spheres NF (Celphere CP-305) | Beads | 968.05 | 24.201 |
| Final Total: Linaclotide Beads, 600 µg/225 mg) | Active beads | 1000 | 25.000 |

### Example 5: Administration of Linaclotide for the treatment of chronic constipation.

Linaclotide Capsules from Example 4 were administered in multicenter, randomized, double-blind, placebo-controlled trials.

### Chronic Constipation Trial 1:

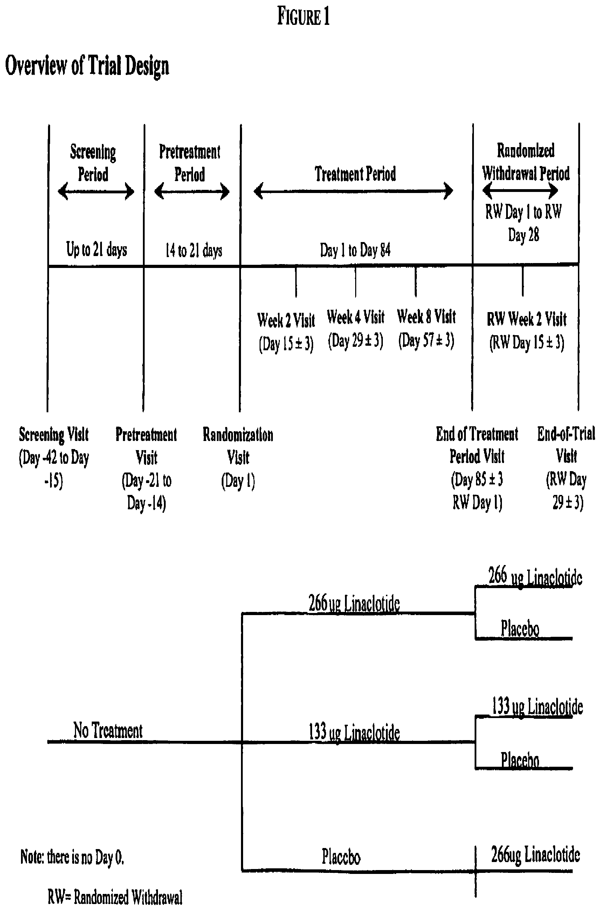

Trial 1 was conducted in 630 patients meeting modified Rome II criteria for chronic constipation. The trial included a two-week pretreatment baseline period and a 12-week treatment period. A diagram of the study design for Trial 2 is given in Figure 1, which is substantially similar to Trial 1 except for the addition of a Randomized Withdrawal period in Trial 2 as described below.

### Pretreatment (Baseline) Period:

The Pretreatment Period is defined as the 14 to 21 calendar days immediately before starting the trial during which where patients provided information related to their daily bowel habits, daily assessment of the symptom severity, constipation severity, and use of other medicines, laxatives, suppositories, and/or enemas. Patients who satisfy the necessary criteria were entered into the Treatment Period. During the two-week pretreatment period, 72 percent of patients in Trial 1 had no CSBMs. A CSBM is a bowel movement that occurs in the absence of laxative, enema, or suppository usage within the preceding 24 hours that is accompanied by the patient self-reporting a feeling of complete emptying of the bowel.

### Treatment Period:

The Treatment Period began with randomization and lasted for 12 weeks. Patients were randomized to treatment with 133 µg linaclotide, 266 µg linaclotide, or placebo (1:1:1), taken once daily in the morning. Patients continued to provide their daily assessments such as their daily bowel habit assessments and daily patient symptom severity assessments.

The primary efficacy endpoint was a 12-week CSBM overall responder, a patient who had a three or more CSBMs per week and an increase of at least one CSBM per week over baseline for at least nine of the 12 weeks of the treatment period.

During the two-week pretreatment period, 72 percent of patients had no CSBMs.

### Results of Trial 1:

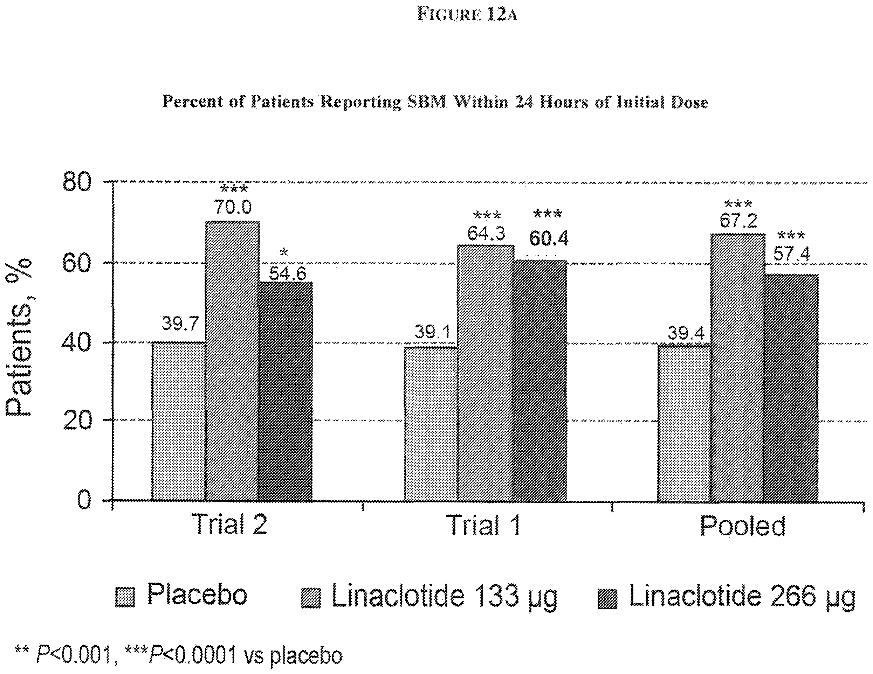

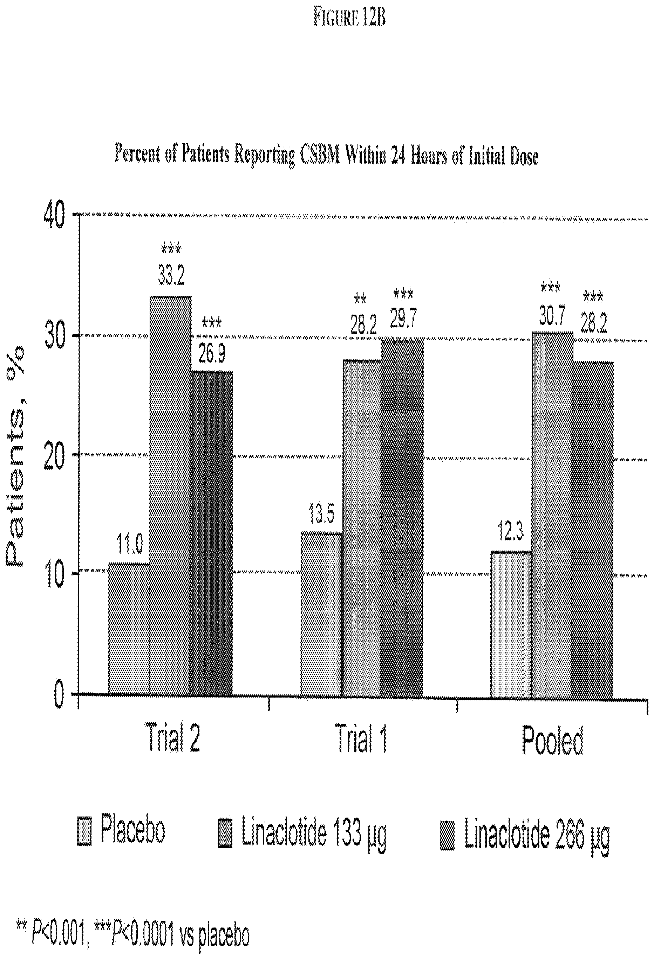

A significantly greater percentage of patients treated with linaclotide reported a SBM or CSBM within 24 hours of treatment than patients in the placebo group (Figures 12a and 12b). A total of 64.3 percent of patients in the 133 µg linaclotide treatment group reported at least one SBM within 24 hours of treatment (p<0.0001), and 60.4 percent reported at least one SBM within 24 hours of treatment in the 266 µg group (p<0.0001), versus 39.1 percent reporting at least one SBM in the placebo group. A total of 28.2 percent of patients in the 133 µg linaclotide treatment group reported at least one CSBM within 24 hours of treatment (p<0.001), and 29.7 percent reported at least one CSBM within 24 hours of treatment in the 266 µg group (p<0.0001), versus 13.5 percent reporting at least one SBM in the placebo group.

After one week, statistically significant improvements from baseline to Week 1 for the 133 µg and 266 µg linaclotide groups versus the change over baseline in the placebo were observed for SBM rate, CSBM rate, stool consistency, constipation severity, and straining (all at p<0.0001); bloating (p<0.01 for 133 µg, p<0.0001 for 266 µg), and abdominal discomfort (p<0.001 for 133 µg, p<0.0001 for 266 µg). The mean changes from baseline for each variable in each treatment group are provided in Table 4, below.

**Table 4: Week 1 Mean Change from Baseline in CC symptoms, by Dose**

| | **Placebo** | | | **133 µg Linaclotide** | | | **266 µg Linaclotide** | | |
|---|---|---|---|---|---|---|---|---|---|
| | **Trial** 2 **(n=209)** | **Trial** 1 **(n=215)** | **Pooled (n=424)** | **Trial 2 (n=217)** | **Trial 1 (n=213)** | **Pooled (n=430)** | **Trial 2 (n=216)** | **Trial 1 (n=202)** | **Pooled (n=418)** |
| **SBM rate** | 1.11 | 1.19 | 1.31 | 3.59*** | 4.09*** | 3.96*** | 3.61*** | 4.09*** | 4.01*** |
| **CSBM rate** | 0.24 | 0.45 | 0.48 | 1.89*** | 1.94*** | 2.02** | 2.02*** | 2.63*** | 2.42** |
| **Stool Consistency^{a}** | 0.33 | 0.38 | 0.39 | 1.75*** | 1.79*** | 1.78*** | 1.81*** | 1.96*** | 1.87*** |
| **Straining^{b}** | -0.28 | -0.38 | -0.33 | -0.99*** | -1.04*** | -1.02*** | -1.06*** | -1.10*** | -1.14*** |
| **Abdominal discomfort^{c}** | -0.13 | -0.06 | -0.12 | -0.23 | -0.27** | -0.26*** | -0.21 | -0.28*** | -0.26*** |
| **Bloating^{c}** | -0.13 | -0.14 | -0.13 | -0.32** | -0.31* | -0.31*** | -0.27* | -0.36*** | -0.31*** |
| **Constipation severity^{c}** | -0.22 | -0.13 | -0.23 | -0.80*** | -0.78*** | -0.82*** | -0.76*** | -0.81*** | -0.85*** |

| | | | | | | | | | |
|---|---|---|---|---|---|---|---|---|---|
| *P<0.01, **P<0.001, *** P<0.0001 vs placebo ^{a} 7-point scale (BSFS): 1 = separate hard lumps like nuts; 7 = watery, no solid pieces. ^{b} 5-point scale: 1 = none; 5 = an extreme amount. ^{c} 5-point scale: 1 = none; 5 = very severe. | | | | | | | | | |

The 12-week CSBM overall responder rate was 16.0 percent in the 133 µg linaclotide group (p=0.0012) and 21.3 percent in the 266 µg linaclotide group (p≤0.0001), a numerical increase of 2.6 and 3.5 fold, respectively, as compared to 6.0 percent in the placebo group. A total of 16.0 percent (p=0.0012) of patients receiving 133 µg and 21.8 percent (p≤0.0001) of patients receiving 266 µg of linaclotide experienced ≥3 weekly CSBMs in at least nine out of 12 weeks as compared to 6.0 percent of patients receiving placebo. In addition 31.0 percent (p≤0.0001) of patients receiving 133 µg and 40.1 percent (p≤0.0001) of patients receiving 266 µg of linaclotide achieved an increase of ≥1 from baseline in weekly CSBMs in at least nine out of 12 weeks as compared to 13.0 percent of patients receiving placebo.

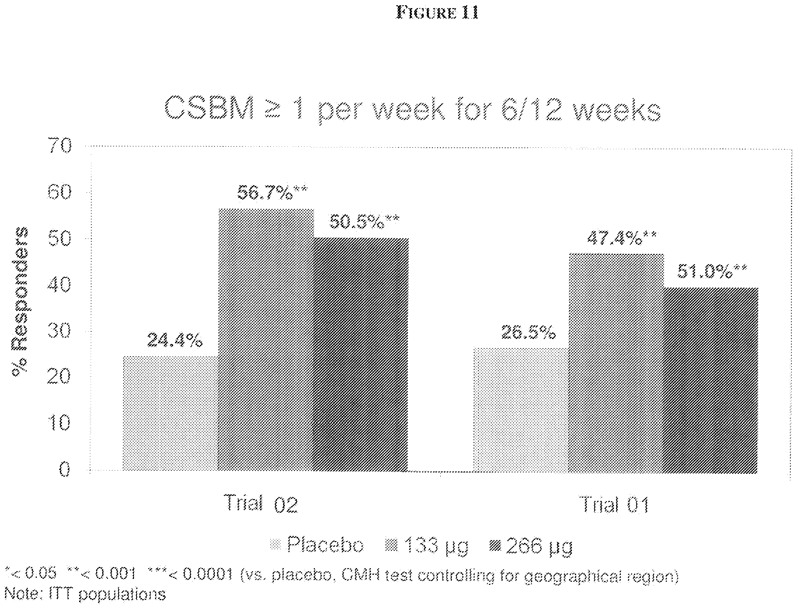

In at least six out of 12 weeks, 47.4 percent (p≤0.001) of patients receiving 133 µg and 51.0 percent (p≤0.001) of patients receiving 266 µg of linaclotide achieved an increase of ≥1 from baseline in weekly CSBMs as compared to 26.5 percent of patients receiving placebo (Figure 11).

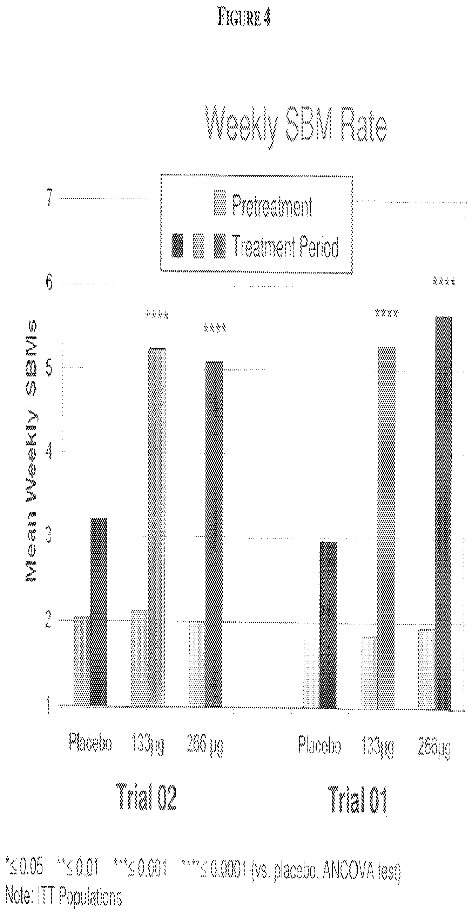

Linaclotide-treated patients demonstrated a significant increase in average weekly CSBMs from baseline (0.6 for placebo; 2.0 for 133 µg, p≤0.0001; 2.7 for 266 µg, p≤0.0001). Linaclotide-treated patients demonstrated a significant increase in average weekly SBMs from baseline (1.1 for placebo; 3.4 for 133 µg, p≤0.0001; 3.7 for 266 µg, p≤0.0001) (Figure 4).

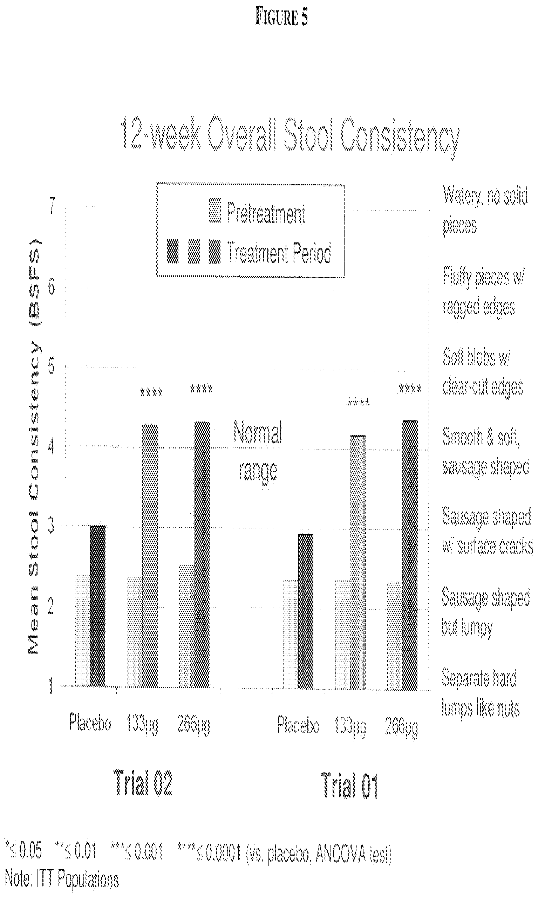

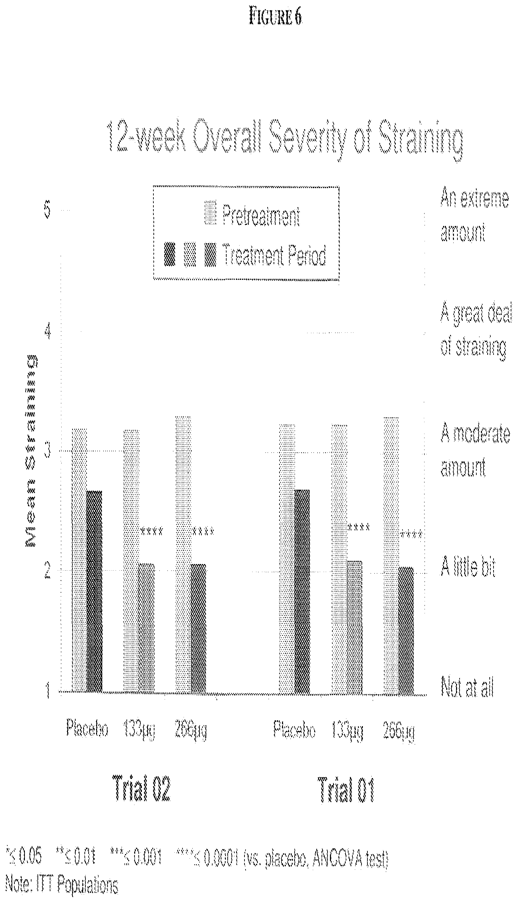

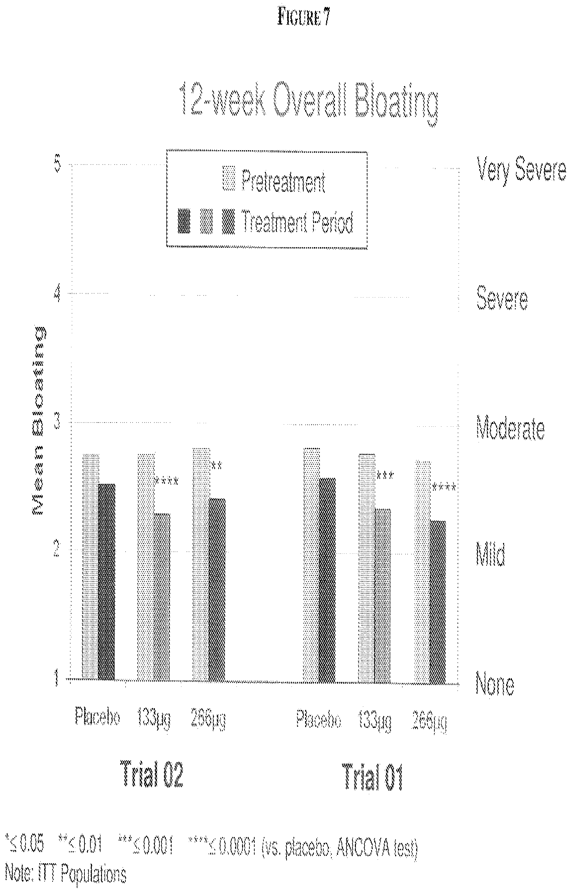

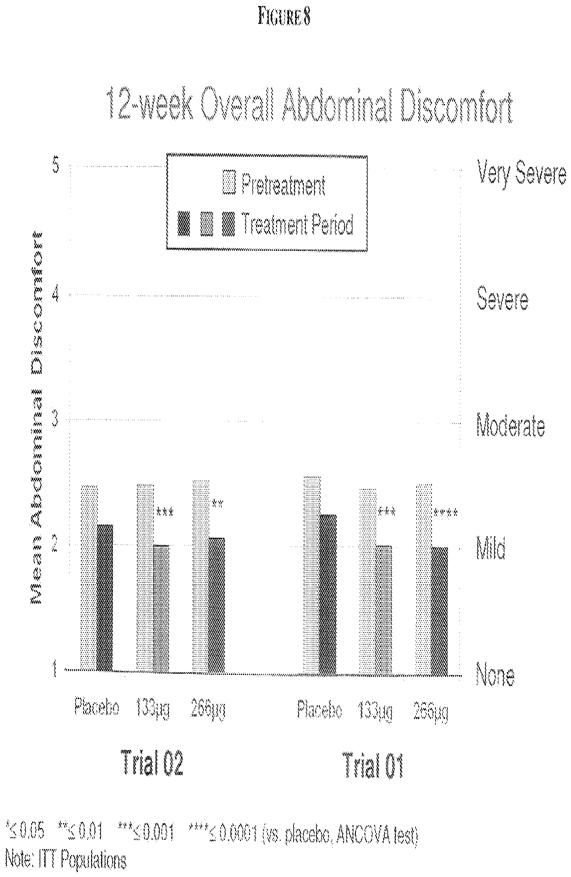

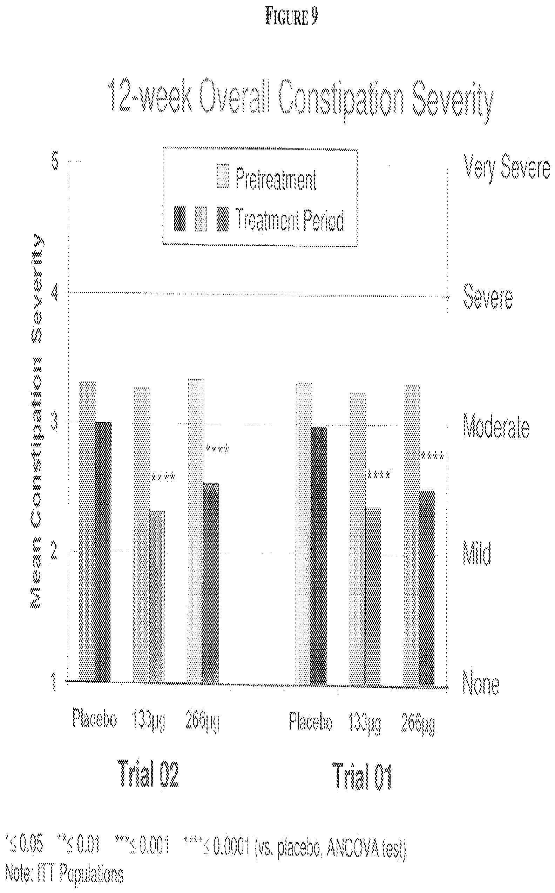

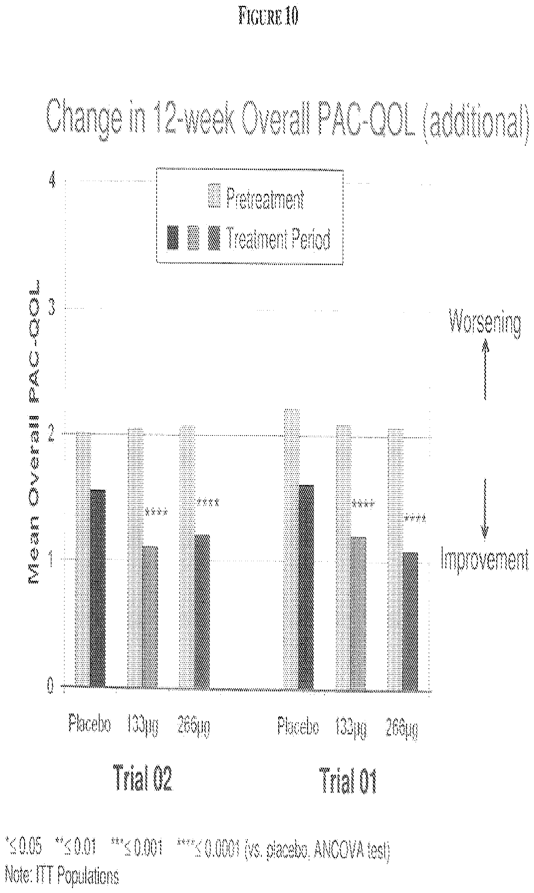

The patients in Trial 1 also exhibited qualitative improvements for the 12 week overall mean changes for linaclotide-treated patients versus the placebo-treated patients (Figures 5-10). These patients exhibited statistically significant improvements in scores for: stool consistency for both doses of 133 µg (p≤0.0001) and 266 µg (p≤0.0001) (Figure 5); severity of straining for both doses of 133 µg (p≤0.0001) and 266 µg (p≤0.0001) (Figure 6); bloating for both doses of 133 µg (p≤0.001) and 266 µg (p≤0.0001) (Figure 7); abdominal discomfort across for doses of 133 µg (p≤0.001) and 266 µg (p≤0.0001) (Figure 8); constipation severity for both doses of 133 µg (p≤0.001) and 266 µg (p≤0.0001) (Figure 9); and PAC-QOL overall assessments at both doses of 133 µg (p≤0.0001) and 266 µg (p≤0.0001) (Figure 10).

Patients in Trial 1 receiving linaclotide treatment also had significant qualitative improvements in PAC-QOL subscales scores for Satisfaction, Physical Discomfort, and Worries/Concerns Subscale. These patients exhibited statistically significant improvements for linaclotide versus placebo for subscale scores for (i) Satisfaction across both doses of 133 µg (p≤0.0001) and 266 µg (p≤0.0001), (ii) Physical Discomfort across both doses of 133 µg (p≤0.0001) and 266 µg (p≤0.0001), and (iii) Worries/Concerns across both doses of 133 µg (p≤0.0001) and 266 µg (p≤0.0001).

### Chronic Constipation Trial 2:

Trial 2 was conducted in 642 patients and was identical to Trial 1 except that Trial 2 also included a four-week Randomized Withdrawal Period, defined below. The experimental design for Trial 2 is given in Figure 1. During the two-week pretreatment (baseline) period, 68 percent of patients in Trial 2 had no CSBMs.

### Results of Trial 2:

A significantly greater percentage of patients treated with linaclotide reported a SBM or CSBM within 24 hours of treatment than patients in the placebo group (Figures 12a and 12b). A total of 70.0 percent of patients in the 133 µg linaclotide treatment group reported at least one SBM within 24 hours of treatment (p<0.0001), and 54.6 percent reported at least one SBM within 24 hours of treatment in the 266 µg group (p<0.01), versus 39.7 percent reporting at least one SBM in the placebo group. A total of 33.2 percent of patients in the 133 µg linaclotide treatment group reported at least one CSBM within 24 hours of treatment (p<0.0001), and 26.9 percent reported at least one CSBM within 24 hours of treatment in the 266 µg group (p<0.0001), versus 11.0 percent reporting at least one SBM in the placebo group.

After one week, statistically significant improvements from baseline to Week 1 for the 133 µg and 266 µg linaclotide groups versus the change over baseline in the placebo were observed for SBM rate, CSBM rate, stool consistency, constipation severity, and straining (all at p<0.0001); as well as bloating (p<0.001 for 133 µg, p<0.01 for 266 µg). Statistically significant improvement were seen in abdominal discomfort in week 2 of study 2 (p<0.01). The mean changes from baseline for each variable in each treatment group are provided in Table 4, above.

The 12-week CSBM overall responder rate was 21.2 percent in the 133 µg linaclotide group (p≤0.0001) and 19.4 percent in the 266 µg linaclotide group (p≤0.0001), a numerical increase of 6.3 and 5.8 fold, respectively, as compared to 3.3 percent in the placebo group. A total of 21.7 percent (p≤0.0001) of patients receiving 133 µg and 19.4 percent (p≤0.0001) of patients receiving 266 µg of linaclotide experienced ≥3 weekly CSBMs in at least nine out of 12 weeks as compared to 3.8 percent of patients receiving placebo. In addition 39.2 percent (p≤0.0001) of patients receiving 133 µg and 37.0 percent (p≤0.0001) of patients receiving 266 µg of linaclotide achieved an increase of ≥1 from baseline in weekly CSBMs in at least nine out of 12 weeks as compared to 11.0 percent of patients receiving placebo.

In at least six out of 12 weeks 56.7 percent (p≤0.001) of patients receiving 133 µg and 50.5 percent (p≤0.001) of patients receiving 266 µg of linaclotide achieved an increase of ≥1 from baseline in weekly CSBMs as compared to 24.4 percent of patients receiving placebo (Figure 11).

Linaclotide-treated patients demonstrated a significant increase in average weekly CSBMs from baseline (0.5 for placebo; 1.9 for 133 µg, p≤0.0001; 2.0 for 266 µg, p≤0.0001). Linaclotide-treated patients demonstrated a significant increase in average weekly SBMs from baseline (1.1 for placebo; 3.0 for 133 µg, p≤0.0001; 3.0 for 266 µg, p≤0.0001) (Figure 4).

The patients in Trial 2 also exhibited qualitative improvements in overall change for bloating (p≤0.01 and p≤0.001), abdominal discomfort (p≤0.01 and p≤0.001), overall stool consistency (p≤0.001), overall severity in straining (p≤0.001), and constipation severity (p≤0.001), which were statistically significant for linaclotide versus placebo for both doses.

The patients in Trial 2 also exhibited qualitative improvements for the 12 week overall mean changes for linaclotide-treated patients versus the placebo-treated patients (Figures 5-10). These patients exhibited statistically significant improvements in scores for: stool consistency for both doses of 133 µg (p≤0.0001) and 266 µg (p≤0.0001); severity of straining for both doses of 133 µg (p≤0.0001) and 266 µg (p≤0.0001); bloating for both doses of 133 µg (p≤0.0001) and 266 µg (p≤0.01); abdominal discomfort across for doses of 133 µg (p≤0.001) and 266 µg (p≤0.01); overall constipation severity for both doses of 133 µg (p≤0.0001) and 266 µg (p≤0.0001); and PAC-QOL overall assessments at both doses of 133 µg (p≤0.0001) and 266 µg (p≤0.0001).

Patients in Trial 1 receiving linaclotide treatment also had significant qualitative improvements in PAC-QOL subscales scores for Satisfaction, Physical Discomfort, and Worries/Concerns Subscale. These patients exhibited statistically significant improvements for linaclotide versus placebo for subscale scores for (i) Satisfaction across both doses of 133 µg (p≤0.0001) and 266 µg (p≤0.0001), (ii) Physical Discomfort across both doses of 133 µg (p≤0.0001) and 266 µg (p≤0.0001), and (iii) Worries/Concerns across both doses of 133 µg (p≤0.0001) and 266 µg (p≤0.0001).

### Randomized Withdrawal Period:

The Randomized Withdrawal (RW) Period is defined as the 4 weeks immediately following the Treatment Period. The beginning of the RW Period coincides with the end of the Treatment Period. Patients who were randomized to linaclotide in the Treatment Period and complete the 12 weeks of the Treatment Period were randomized to treatment with linaclotide or placebo in the RW Period. Patients who were randomized to placebo in the Treatment Period and complete the 12 weeks of the Treatment Period received 133µg or 266µg linaclotide in the RW Period.

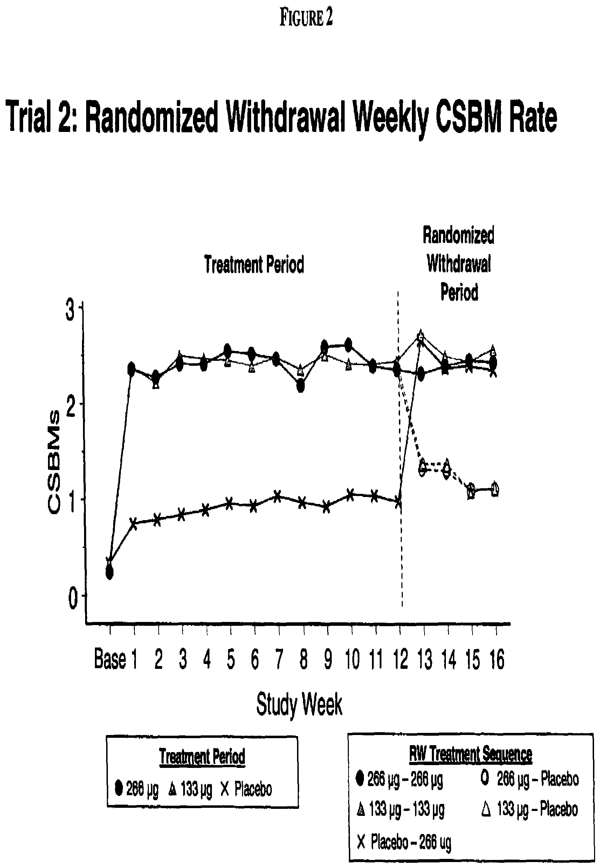

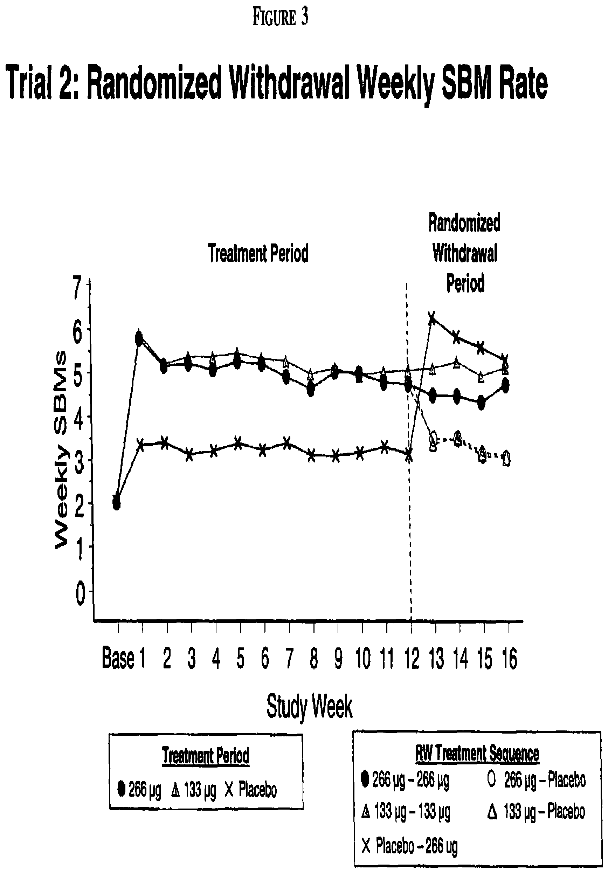

The results from the Randomized Withdrawal Period are shown below in Table 5 and Figures 2 and 3 and demonstrate that for (i) patients administered linaclotide for the 12 week period and re-randomized to placebo during the 4 week withdrawal period there were no decreases in CSBM (Figure 2) or SBM rates (Figure 3) below the baseline rates for these patients, and (ii) patients administered placebo for the 12 week period and re-randomized to linaclotide during the 4 week withdrawal period exhibited CSBM/SBM rates and qualitative improvements in other bowel and abdominal symptoms similar to the rates and improvements experienced by patients treated for 12 weeks with linaclotide (see Figures 2 and 3).

The results from Randomized Withdrawal Period additionally demonstrate that patients initiating linaclotide treatment had marked improvement in CC symptoms. There was no evidence of rebound of CC symptoms or increase in the frequencies of adverse events following discontinuing of linaclotide treatment. The incidence of adverse events in patients initiating linaclotide treatment was similar to the incidence in those receiving linaclotide during the first 4 weeks of the Treatment Period. Patients continuing linaclotide treatment showed sustained improvements in bowel and abdominal symptoms and global assessments.

**Table 5: Change From Baseline in Bowel and Abdominal Symptoms (RW Period, by Treatment Sequence; RW Population)**

| **Efficacy Parameter** | **Baseline Value Mean (SD)** | **RW: Placebo Mean (95% CI)** | | **RW: Linaclotide Mean (95% CI)** | | |
|---|---|---|---|---|---|---|
| | **All Patients (N=538)** | **133 µg/Placebo (n=95)** | **266 µg/Placebo (n=86)** | **133 µg/133 µg (n=90)** | **266 µg/266 µg (n=90)** | **Placebo/266 µg (n=177)** |
| **Weekly CSBM Rate** | 0.3 (0.6) | 1.0 (0.6, 1.3) | 0.9 (0.5, 1.4) | 2.1 (1.5,2.8) | 2.2 (1.6,2.8) | 2.1 (1.7,2.5) |
| **Weekly SBM Rate** | 2.0 (1.6) | 1.2 (0.6, 1.8) | 1.3 (0.6, 2.0) | 3.0 (2.3, 3.6) | 2.5 (1.8,3.2) | 3.7 (3.1,4.3) |
| **Stool Consistency (BSFS)** | 2.4 (1.0) | 1.0 (0.7, 1.2) | 0.8 (0.5, 1.1) | 1.9 (1.6,2.3) | 1.6 (1.3, 1.9) | 1.9 (1.6,2.1) |
| **Straining** | 3.2 (0.9) | -0.8 (-1.0, -0.7) | -0.8 (-1.1, -0.5) | -1.1 (-1.3, -0.9) | -1.3 (-1.5, -1.1) | -1.2 (-1.3, -1.0) |
| **Abdominal Discomfort** | 2.5 (0.8) | -0.4 (-0.6, -0.3) | -0.4 (-0.6, -0.2) | -0.5 (-0.7, -0.4) | -0.7 (-0.8, -0.5) | -0.5 (-0.6, -0.4) |
| **Bloating** | 2.8 (0.9) | -0.4 (-0.5, -0.2) | -0.2 (-0.4, -0.3) | -0.4 (-0.6, -0.3) | -0.6 (-0.7, -0.4) | -0.5 (-0.6, -0.3) |
| **Constipation Severity** | 3.3 (0.7) | -0.5 (-0.7, -0.3) | -0.5 (-0.7, -0.3) | -0.9 (-1.1, -0.7) | -0.9 (-1.2, -0.7) | -1.0 (-1.1, -0.9) |
| **Global Relief of Constipation Symptoms** | 4.0 (0.6) | -0.7 (-1.0, -0.5) | -0.8 (-1.1, -0.6) | -1.3 -1.5, -1.1) | -1.3 (-1.6, -1.1) | -1.3 (-1.6, -1.1) |

| | | | | | | |
|---|---|---|---|---|---|---|
| CI = confidence interval; SD = std. deviation | | | | | | |

For both trial 1 and 2, the most common adverse events in linaclotide-treated patients were diarrhea, flatulence, and abdominal pain. Overall rates of discontinuation due to adverse events were 7.4 percent for linaclotide and 4.2 percent for placebo.

### OTHER EMBODIMENTS

Should the meaning of the terms in any of the patents or publications referred to in this disclosure conflict with the meaning of the terms used in this disclosure, the meaning of the terms in this disclosure are intended to be controlling.

## Claims

1. A pharmaceutical composition for use in a method of treating chronic constipation in a patient, wherein the pharmaceutical composition is administered once a day and the pharmaceutical composition is administered in the morning or at least 30 minutes before ingestion of food, and wherein the pharmaceutical composition comprises:
(a) linaclotide or pharmaceutically acceptable salts thereof;
(b) CaCl₂;
(c) L-leucine; and
(d) hydroxypropyl methylcellulose;
wherein linaclotide is present in the pharmaceutical composition in an amount between 100 µg to 600 µg and the molar ratio of Ca²⁺:leucine:linaclotide is between 5-100:5-50:1.

2. The pharmaceutical composition for use of any of claim 1, wherein linaclotide is present in the pharmaceutical composition in an amount of about 150 µg or about 300 µg.

3. The pharmaceutical composition for use of claim 1 or claim 2, wherein the pharmaceutical composition is administered for a period of greater than four weeks, at least 6 weeks or at least 12 weeks.

4. The pharmaceutical composition for use of any of claims 1-3, wherein the pharmaceutical composition is administered at least once a week, at least twice a week, at least three times a week, at least four times a week, at least five times a week, at least six times a week or each day of the week.

5. The pharmaceutical composition for use of any of claims 1-4, wherein the pharmaceutical composition is provided as a capsule or tablet.

6. The pharmaceutical composition for use of any of claims 1-5, wherein administration of the pharmaceutical composition increases the number of complete spontaneous bowel movements (CSBMs) by the patient to three or greater CSBMs per week or by at least one CSBM per week compared to said patient prior to administration of the pharmaceutical composition.

7. The pharmaceutical composition for use of any of claims 1-6, wherein administration of the pharmaceutical composition decreases abdominal bloating, abdominal discomfort, constipation severity or straining during defecation in said patient compared to said patient prior to administration of the pharmaceutical composition.

8. The pharmaceutical composition for use of any of claims 1-7, wherein administration of the pharmaceutical composition improves stool consistency or patient assessment of constipation quality of life compared to said patient prior to administration of the pharmaceutical composition.

9. The pharmaceutical composition for use of any of claims 1-8, wherein discontinuing the administration of the pharmaceutical composition does not produce a rebound of a symptom in said patient, wherein said symptom is selected from a decrease in the number of CSBMs per week, a decrease in the number of spontaneous bowel movements (SBMs) per week, an increase in bloating, an increase in abdominal discomfort, an increase in constipation severity, a decrease in stool consistency, an increase in straining during defecation, and a decrease in global relief of constipation for said patient.

10. The pharmaceutical composition for use of any of claims 1-8, wherein administration of the pharmaceutical composition to said patient improves the SBM rate, CSBM rate, stool consistency, straining, abdominal discomfort, bloating, and constipation severity within one week after the administration of the pharmaceutical composition.

11. The pharmaceutical composition for use of claim 10, wherein the patient exhibits an improvement in SBM rate, CSBM rate, stool consistency, straining, abdominal discomfort, bloating, and constipation severity within 72 hours, 48 hours, or 24 hours after administration of the pharmaceutical composition.

12. The pharmaceutical composition for use of any of claims 1-8, wherein administration of the pharmaceutical composition produces a rapid or sustained relief of symptoms associated with chronic constipation.

13. The pharmaceutical composition for use of claim 12, wherein the rapid relief occurs within one week, 72 hours, 48 hours, or 24 hours.

14. The pharmaceutical composition for use of claim 12, wherein the sustained relief occurs for at least 16 weeks; at least 9 weeks out of 16 weeks; at least 6 weeks out of 16 weeks; at least 3 weeks out of 4 weeks; at least 1 week out of 2 weeks; at least 1 week; or at least 2 weeks.

15. The pharmaceutical composition for use of any of claims 1-14, wherein the method comprises administering a first dose of linaclotide once a day and, after one or more days of administering said first dose, administering a second dose of linaclotide once a day, wherein said second dose is lower than said first dose and wherein at least one of said first and second doses is provided as a said pharmaceutical composition.

16. The pharmaceutical composition for use of any of claims 1-14, wherein the method comprises administering a first dose of linaclotide once a day on a first schedule and, after one or more days of administering said first dose, administering a second dose of linaclotide on a second schedule that is less frequent than said first schedule, wherein at least one of said first and second doses is provided as a said pharmaceutical composition.

17. The pharmaceutical composition for use of claim 16, wherein the first dose is the same as the second dose of linaclotide.

18. The pharmaceutical composition for use of claim 16, wherein the first dose and the second dose are each about 300 µg or about 150 µg.

19. The pharmaceutical composition for use of claim 16, wherein the second dose is lower than the first dose.

20. The pharmaceutical composition for use of claim 16, wherein said second schedule comprises administration of said second dose of linaclotide every other day, every third day, every fourth day, every fifth day, every sixth day or once weekly.

21. The pharmaceutical composition for use of any of claims 1-14, wherein the method comprises administering a first dose of linaclotide once a day and, after one or more days of administering said first dose, administering a second dose of linaclotide once a day, wherein said second dose is higher than said first dose and wherein at least one of said first and second doses is provided as a said pharmaceutical composition.

22. The pharmaceutical composition for use of claim 21, wherein the first dose is about 150 µg and the second dose is about 300 µg.

23. The pharmaceutical composition for use of any of claims 1-14, wherein the method comprises administering a first dose of linaclotide once a day on a first schedule and, after one or more days of administering said first dose, administering a second dose of linaclotide on a second schedule that is more frequent than said first schedule, wherein at least one of said first and second doses is provided as a said pharmaceutical composition.

24. The pharmaceutical composition for use of claim 23, wherein said first schedule comprises administration of said first dose of linaclotide every other day, every third day, every fourth day, every fifth day, every sixth day or once weekly.

25. Use of linaclotide for the manufacture of a pharmaceutical composition for the treatment of chronic constipation in a patient, wherein the pharmaceutical composition is administered once a day and the pharmaceutical composition is administered in the morning or at least 30 minutes before ingestion of food, and wherein the pharmaceutical composition comprises:
(a) linaclotide or pharmaceutically acceptable salts thereof;
(b) CaCl₂;
(c) L-leucine; and
(d) hydroxypropyl methylcellulose;
wherein linaclotide is present in the pharmaceutical composition in an amount between 100 µg to 600 µg and the molar ratio of Ca²⁺:leucine:linaclotide is between 5-100:5-50:1.

## Patentansprüche

1. Pharmazeutische Zusammensetzung zur Verwendung in einem Verfahren zur Behandlung von chronischer Verstopfung bei einem Patienten, wobei die pharmazeutische Zusammensetzung einmal täglich verabreicht wird und die pharmazeutische Zusammensetzung morgens oder mindestens 30 Minuten vor Nahrungsaufnahme verabreicht wird, und wobei die pharmazeutische Zusammensetzung Folgendes enthält:
a) Linaclotid oder pharmazeutisch verträgliche Salze davon;
b) CaCl₂;
c) L-Leucin; und
d) Hydroxypropylmethylcellulose;
wobei Linaclotid in der pharmazeutischen Zusammensetzung in einer Menge zwischen 100 µg und 600 µg vorliegt und das Molverhältnis von Ca²⁺:Leucin:Linaclotid zwischen 5-100:5-50:1 liegt.

2. Pharmazeutische Zusammensetzung zur Verwendung nach einem von Anspruch 1, wobei Linaclotid in der pharmazeutischen Zusammensetzung in einer Menge von ca. 150 µg oder ca. 300 µg vorliegt.

3. Pharmazeutische Zusammensetzung zur Verwendung nach Anspruch 1 oder Anspruch 2, wobei die pharmazeutische Zusammensetzung für eine Dauer von mehr als vier Wochen, mindestens 6 Wochen oder mindestens 12 Wochen verabreicht wird.

4. Pharmazeutische Zusammensetzung zur Verwendung nach einem der Ansprüche 1 bis 3, wobei die pharmazeutische Zusammensetzung mindestens einmal wöchentlich, mindestens zweimal wöchentlich, mindestens dreimal wöchentlich, mindestens viermal wöchentlich, mindestens fünfmal wöchentlich, mindestens sechsmal wöchentlich oder jeden Tag in der Woche verabreicht wird.

5. Pharmazeutische Zusammensetzung zur Verwendung nach einem der Ansprüche 1 bis 4, wobei die pharmazeutische Zusammensetzung als eine Kapsel oder Tablette vorgesehen ist.

6. Pharmazeutische Zusammensetzung zur Verwendung nach einem der Ansprüche 1 bis 5, wobei die Verabreichung der pharmazeutischen Zusammensetzung die Anzahl an vollständigen spontanen Darmentleerungen (CSBMs, complete spontaneous bowel movements) des Patienten auf drei oder mehr CSBMs pro Woche oder um mindestens eine CSBM pro Woche erhöht, verglichen mit dem genannten Patienten vor Verabreichung der pharmazeutischen Zusammensetzung.

7. Pharmazeutische Zusammensetzung zur Verwendung nach einem der Ansprüche 1 bis 6, wobei die Verabreichung der pharmazeutischen Zusammensetzung das abdominelle Völlegefühl, abdominelle Beschwerden, den Schweregrad der Verstopfung oder das Pressen während der Defäkation bei dem genannten Patienten vermindert, verglichen mit dem genannten Patienten vor Verabreichung der pharmazeutischen Zusammensetzung.

8. Pharmazeutische Zusammensetzung zur Verwendung nach einem der Ansprüche 1 bis 7, wobei die Verabreichung der pharmazeutischen Zusammensetzung die Stuhlkonsistenz oder die Beurteilung der Lebensqualität des Patienten mit Verstopfung verbessert, verglichen mit dem genannten Patienten vor Verabreichung der pharmazeutischen Zusammensetzung.

9. Pharmazeutische Zusammensetzung zur Verwendung nach einem der Ansprüche 1 bis 8, wobei das Absetzen der Verabreichung der pharmazeutischen Zusammensetzung kein Rebound-Symptom bei dem genannten Patienten produziert, wobei das genannte Symptom ausgewählt ist aus einer Abnahme der Anzahl an CSBMs pro Woche, einer Abnahme der Anzahl an spontanen Darmentleerungen (SBMs, spontaneous bowel movements) pro Woche, einer Zunahme des Völlegefühls, einer Zunahme der abdominellen Beschwerden, einer Zunahme des Schweregrads der Verstopfung, einer Abnahme der Stuhlkonsistenz, einer Zunahme des Pressens während der Defäkation und einer Abnahme der globalen Linderung der Verstopfung bei dem genannten Patienten.

10. Pharmazeutische Zusammensetzung zur Verwendung nach einem der Ansprüche 1 bis 8, wobei die Verabreichung der pharmazeutischen Zusammensetzung an den genannten Patienten die SBM-Rate, die CSBM-Rate, die Stuhlkonsistenz, das Pressen, die abdominellen Beschwerden, das Vollegefühl, den Schweregrad der Verstopfung innerhalb einer Woche nach Verabreichung der pharmazeutischen Zusammensetzung verbessert.

11. Pharmazeutische Zusammensetzung zur Verwendung nach Anspruch 10, wobei der Patient eine Verbesserung der SBM-Rate, der CSBM-Rate, der Stuhlkonsistenz, des Pressens, der abdominellen Beschwerden, des Völlegefühls und des Schweregrads der Verstopfung innerhalb 72 Stunden, 48 Stunden oder 24 Stunden nach Verabreichung der pharmazeutischen Zusammensetzung aufweist.

12. Pharmazeutische Zusammensetzung zur Verwendung nach einem der Ansprüche 1 bis 8, wobei die Verabreichung der pharmazeutischen Zusammensetzung eine rasche oder anhaltende Linderung der mit der chronischen Verstopfung assoziierten Symptome bewirkt.

13. Pharmazeutische Zusammensetzung zur Verwendung nach Anspruch 12, wobei die rasche Linderung innerhalb einer Woche, 72 Stunden, 48 Stunden oder 24 Stunden auftritt.

14. Pharmazeutische Zusammensetzung zur Verwendung nach Anspruch 12, wobei die anhaltende Linderung für mindestens 16 Wochen; mindestens 9 Wochen von 16 Wochen; mindestens 6 Wochen von 16 Wochen; mindestens 3 Wochen von 4 Wochen; mindestens eine Woche von 2 Wochen; mindestens 1 Woche; oder mindestens 2 Wochen auftritt.

15. Pharmazeutische Zusammensetzung zur Verwendung nach einem der Ansprüche 1 bis 14, wobei das Verfahren Folgendes umfasst: Verabreichen einer ersten Linaclotid-Dosis einmal täglich und nach einem Tag oder mehr Tagen des Verabreichens der genannten ersten Dosis Verabreichen einer zweiten Linaclotid-Dosis einmal täglich, wobei die genannte zweite Dosis niedriger ist als die genannte erste Dosis und wobei mindestens eine der genannten ersten und zweiten Dosen als eine genannte pharmazeutische Zusammensetzung vorgesehen ist.

16. Pharmazeutische Zusammensetzung zur Verwendung nach einem der Ansprüche 1 bis 14, wobei das Verfahren Folgendes umfasst: Verabreichen einer ersten Linaclotid-Dosis einmal täglich unter einem ersten Plan und nach einem Tag oder mehr Tagen des Verabreichens der genannten ersten Dosis Verabreichen einer zweiten Linaclotid-Dosis unter einem zweiten Plan, der weniger frequent ist als der genannte erste Plan, wobei mindestens eine der genannten ersten und zweiten Dosen als eine genannte pharmazeutische Zusammensetzung vorgesehen ist.

17. Pharmazeutische Zusammensetzung zur Verwendung nach Anspruch 16, wobei die erste Dosis die gleiche wie die zweite Linaclotid-Dosis ist.

18. Pharmazeutische Zusammensetzung zur Verwendung nach Anspruch 16, wobei die erste Dosis und die zweite Dosis jeweils ca. 300 µg oder ca. 150 µg betragen.

19. Pharmazeutische Zusammensetzung zur Verwendung nach Anspruch 16, wobei die zweite Dosis niedriger als die erste Dosis ist.

20. Pharmazeutische Zusammensetzung zur Verwendung nach Anspruch 16, wobei der genannte zweite Plan die Verabreichung der genannten zweiten Linaclotid-Dosis jeden zweiten Tag, jeden dritten Tag, jeden vierten Tag, jeden fünften Tag, jeden sechsten Tag oder einmal wöchentlich umfasst.

21. Pharmazeutische Zusammensetzung zur Verwendung nach einem der Ansprüche 1 bis 14, wobei das Verfahren Folgendes umfasst: Verabreichen einer ersten Linaclotid-Dosis einmal täglich und nach einem oder mehr Tagen des Verabreichens der genannten ersten Dosis Verabreichen einer zweiten Linaclotid-Dosis einmal täglich, wobei die genannte zweite Dosis höher ist als die genannte erste Dosis und wobei mindestens eine der genannten ersten und zweiten Dosen als eine genannte pharmazeutische Zusammensetzung vorgesehen ist.

22. Pharmazeutische Zusammensetzung zur Verwendung nach Anspruch 21, wobei die erste Dosis ca. 150 µg beträgt und die zweite Dosis ca. 300 µg beträgt.

23. Pharmazeutische Zusammensetzung zur Verwendung nach einem der Ansprüche 1 bis 14, wobei das Verfahren Folgendes umfasst: Verabreichen einer ersten Linaclotid-Dosis einmal täglich unter einem ersten Plan und nach einem Tag oder mehr Tagen des Verabreichens der genannten ersten Dosis Verabreichen einer zweiten Linaclotid-Dosis unter einem zweiten Plan, der frequenter ist als der genannte erste Plan, wobei mindestens eine der genannten ersten und zweiten Dosen als eine genannte pharmazeutische Zusammensetzung vorgesehen ist.

24. Pharmazeutische Zusammensetzung zur Verwendung nach Anspruch 23, wobei der genannte erste Plan die Verabreichung der genannten ersten Linaclotid-Dosis jeden zweiten Tag, jeden dritten Tag, jeden vierten Tag, jeden fünften Tag, jeden sechsten Tag oder einmal wöchentlich umfasst.

25. Verwendung von Linaclotid zur Herstellung einer pharmazeutischen Zusammensetzung zur Behandlung von chronischer Verstopfung bei einem Patienten, wobei die pharmazeutische Zusammensetzung einmal täglich verabreicht wird und die pharmazeutische Zusammensetzung morgens oder mindestens 30 Minuten vor Nahrungsaufnahme verabreicht wird, und wobei die pharmazeutische Zusammensetzung Folgendes enthält:
a) Linaclotid oder pharmazeutisch verträgliche Salze davon;
b) CaCl₂;
c) L-Leucin; und
d) Hydroxypropylmethylcellulose;
wobei Linaclotid in der pharmazeutischen Zusammensetzung in einer Menge zwischen 100 µg bis 600 µg vorliegt und das Molverhältnis von Ca²⁺:Leucin:Linaclotid zwischen 5-100:5-50:1 liegt.

## Revendications

1. Composition pharmaceutique destinée à une utilisation dans une méthode de traitement d'une constipation chronique chez un patient, la composition pharmaceutique étant administrée une fois par jour et la composition pharmaceutique étant administrée le matin ou au moins 30 minutes avant l'ingestion d'aliments, et la composition pharmaceutique comprenant :
(a) du linaclotide ou des sels pharmaceutiquement acceptables de celui-ci ;
(b) du CaCl₂ ;
(c) de la L-leucine ; et
(d) de l'hydroxypropylméthylcellulose ;
le linaclotide étant présent dans la composition pharmaceutique dans une quantité de 100 µg à 600 µg et le rapport molaire Ca²⁺:leucine:linaclotide étant de 5-100:5-50:1.

2. Composition pharmaceutique destinée à une utilisation selon la revendication 1, le linaclotide étant présent dans la composition pharmaceutique dans une quantité d'environ 150 µg ou d'environ 300 µg.

3. Composition pharmaceutique destinée à une utilisation selon la revendication 1 ou la revendication 2, la composition pharmaceutique étant administrée pendant une période de plus de quatre semaines, d'au moins 6 semaines ou d'au moins 12 semaines.

4. Composition pharmaceutique destinée à une utilisation selon l'une quelconque des revendications 1 à 3, la composition pharmaceutique étant administrée au moins une fois par semaine, au moins deux fois par semaine, au moins trois fois une semaine, au moins quatre fois une semaine, au moins cinq fois une semaine, au moins six fois par semaine, ou chaque jour de la semaine.

5. Composition pharmaceutique destinée à une utilisation selon l'une quelconque des revendications 1 à 4, la composition pharmaceutique étant fournie sous la forme d'une capsule ou d'un comprimé.

6. Composition pharmaceutique destinée à une utilisation selon l'une quelconque des revendications 1 à 5, l'administration de la composition pharmaceutique augmentant le nombre de défécations spontanées complètes (DSC) par le patient jusqu'à trois DSC ou plus par semaine, ou d'au moins une DSC par semaine par comparaison avec ledit patient avant l'administration de la composition pharmaceutique.

7. Composition pharmaceutique destinée à une utilisation selon l'une quelconque des revendications 1 à 6, l'administration de la composition pharmaceutique diminuant les ballonnements abdominaux, l'inconfort abdominal, la sévérité de la constipation ou la difficulté à évacuer les selles chez ledit patient par comparaison avec ledit patient avant l'administration de la composition pharmaceutique.

8. Composition pharmaceutique destinée à une utilisation selon l'une quelconque des revendications 1 à 7, l'administration de la composition pharmaceutique améliorant la consistance des selles ou l'évaluation par le patient de sa qualité de vie en ce qui concerne la constipation par comparaison avec ledit patient avant l'administration de la composition pharmaceutique.

9. Composition pharmaceutique destinée à une utilisation selon l'une quelconque des revendications 1 à 8, l'interruption de l'administration de la composition pharmaceutique ne produisant pas de rebond d'un symptôme chez ledit patient, ledit symptôme étant sélectionné parmi une diminution du nombre de DSC par semaine, une diminution du nombre de défécations spontanées (DS) par semaine, une augmentation des ballonnements, une augmentation de l'inconfort abdominal, une augmentation de la sévérité de la constipation, une diminution de la consistance des selles, une augmentation de la difficulté à évacuer les selles, et une diminution du soulagement global de la constipation pour ledit patient.

10. Composition pharmaceutique destinée à une utilisation selon l'une quelconque des revendications 1 à 8, l'administration de la composition pharmaceutique audit patient améliorant le taux de DS, le taux de DSC, la consistance des selles, la difficulté à évacuer les selles, l'inconfort abdominal, les ballonnements, et la sévérité de la constipation, dans un délai d'une semaine après l'administration de la composition pharmaceutique.

11. Composition pharmaceutique destinée à une utilisation selon la revendication 10, le patient présentant une amélioration du taux de DS, du taux de DSC, de la consistance des selles, de la difficulté à évacuer les selles, de l'inconfort abdominal, des ballonnements, et de la sévérité de la constipation, dans un délai de 72 heures, de 48 heures ou de 24 heures après l'administration de la composition pharmaceutique.

12. Composition pharmaceutique destinée à une utilisation selon l'une quelconque des revendications 1 à 8, l'administration de la composition pharmaceutique produisant un soulagement rapide ou prolongé des symptômes associés à la constipation chronique.

13. Composition pharmaceutique destinée à une utilisation selon la revendication 12, le soulagement rapide se produisant dans un délai d'une semaine, de 72 heures, de 48 heures, ou de 24 heures.

14. Composition pharmaceutique destinée à une utilisation selon la revendication 12, le soulagement prolongé se produisant pendant au moins 16 semaines ; au moins 9 semaines sur 16 semaines ; au moins 6 semaines sur 16 semaines ; au moins 3 semaines sur 4 semaines ; au moins 1 semaine sur 2 semaines ; au moins 1 semaine ; ou au moins 2 semaines.

15. Composition pharmaceutique destinée à une utilisation selon l'une quelconque des revendications 1 à 14, la méthode comprenant l'administration d'une première dose de linaclotide une fois par jour et, après un ou plusieurs jours d'administration de ladite première dose, l'administration d'une deuxième dose de linaclotide une fois par jour, ladite deuxième dose étant inférieure à ladite première dose et au moins l'une desdites première et deuxième doses étant fournie sous forme de ladite composition pharmaceutique.

16. Composition pharmaceutique destinée à une utilisation selon l'une quelconque des revendications 1 à 14, la méthode comprenant l'administration d'une première dose de linaclotide une fois par jour selon un premier programme et, après un ou plusieurs jours d'administration de ladite première dose, l'administration d'une deuxième dose de linaclotide selon un deuxième programme dans lequel la fréquence d'administration est inférieure à celle dudit premier programme, au moins l'une desdites première et deuxième doses étant fournie sous forme de ladite composition pharmaceutique.

17. Composition pharmaceutique destinée à une utilisation selon la revendication 16, la première dose étant identique à la deuxième dose de linaclotide.

18. Composition pharmaceutique destinée à une utilisation selon la revendication 16, la première dose et la deuxième dose étant chacune d'environ 300 µg ou d'environ 150 µg.

19. Composition pharmaceutique destinée à une utilisation selon la revendication 16, dans laquelle la deuxième dose est inférieure à la première dose.

20. Composition pharmaceutique destinée à une utilisation selon la revendication 16, dans laquelle ledit deuxième programme comprend l'administration de ladite deuxième dose de linaclotide tous les deux jours, tous les trois jours, tous les quatre jours, tous les cinq jours, tous les six jours ou une fois par semaine.

21. Composition pharmaceutique destinée à une utilisation selon l'une quelconque des revendications 1 à 14, la méthode comprenant l'administration d'une première dose de linaclotide une fois par jour et, après un ou plusieurs jours d'administration de ladite première dose, l'administration d'une deuxième dose de linaclotide une fois par jour, ladite deuxième dose étant supérieure à ladite première dose et au moins l'une desdites première et deuxième doses étant fournie sous forme de ladite composition pharmaceutique.

22. Composition pharmaceutique destinée à une utilisation selon la revendication 21, la première dose étant d'environ 150 µg et la deuxième dose étant d'environ 300 µg.

23. Composition pharmaceutique destinée à une utilisation selon l'une quelconque des revendications 1 à 14, la méthode comprenant l'administration d'une première dose de linaclotide une fois par jour selon un premier programme et, après un ou plusieurs jours d'administration de ladite première dose, l'administration d'une deuxième dose de linaclotide selon un deuxième programme dans lequel la fréquence d'administration est supérieure à celle dudit premier programme, au moins l'une desdites première et deuxième doses étant fournie sous forme de ladite composition pharmaceutique.

24. Composition pharmaceutique destinée à une utilisation selon la revendication 23, dans laquelle ledit premier programme comprend l'administration de ladite première dose de linaclotide tous les deux jours, tous les trois jours, tous les quatre jours, tous les cinq jours, tous les six jours ou une fois par semaine.

25. Utilisation de linaclotide pour la fabrication d'une composition pharmaceutique pour le traitement d'une constipation chronique chez un patient, la composition pharmaceutique étant administrée une fois par jour et la composition pharmaceutique étant administrée le matin ou au moins 30 minutes avant l'ingestion d'aliments, et la composition pharmaceutique comprenant :
(a) du linaclotide ou des sels pharmaceutiquement acceptables de celui-ci ;
(b) du CaCl₂ ;
(c) de la L-leucine ; et
(d) de l'hydroxypropylméthylcellulose ;
le linaclotide étant présent dans la composition pharmaceutique dans une quantité de 100 µg à 600 µg et le rapport molaire Ca²⁺:leucine:linaclotide étant de 5-100:5-50:1.
